# EUROPEAN PATENT SPECIFICATION

(11) **EP 2 972 340 B1**
(45) Date of publication and mention of the grant of the patent: **16.01.2019**
(21) Application number: 14763428.1
(22) Date of filing: 14.03.2014
(51) Int. Cl.: G01N 33/68

(54) **METHODS FOR ANALYZING BLOOD TO DETECT DISEASES ASSOCIATED WITH ABNORMAL PROTEIN AGGREGATION**
VERFAHREN ZUR ANALYSE VON BLUT ZUM NACHWEIS VON KRANKHEITEN IM ZUSAMMENHANG MIT ABNORMALER PROTEINAGGREGATION
PROCÉDÉ POUR ANALYSER DU SANG AFIN DE DÉTECTER DES MALADIES ASSOCIÉES À UNE AGRÉGATION ANORMALE DE PROTÉINES

(30) Priority: 15.03.2013 US 201313833008
(43) Date of publication of application: 20.01.2016
(73) Proprietor: Stys, Peter, Calgary, Alberta T3Z 3M1 (CA); Tsutsui, Shigeki, Calgary, Alberta T3G 0B9 (CA)
(72) Inventor: Stys, Peter, Calgary, Alberta T3Z 3M1 (CA); Tsutsui, Shigeki, Calgary, Alberta T3G 0B9 (CA)
(74) Representative: reuteler & cie SA
(86) International application number: PCT/CA2014/000250
(87) International publication number: WO 2014/138919

(56) References cited:
- EP-A2- 1 395 833
- WO-A1-97/26537
- WO-A1-2010/144634
- WO-A2-2006/020269
- WO-A2-2012/122236
- US-A1- 2007 054 322
- US-A1- 2008 300 204
- STYREN S D ET AL: "X-34, a fluorescent derivative of Congo red: A novel histochemical stain for Alzheimer's disease pathology", JOURNAL OF HISTOCHEMISTRY AND CYTOCHEMISTRY, HISTOCHEMICAL SOCIETY, NEW YORK, NY, US, vol. 48, no. 9, 1 September 2000 (2000-09-01), pages 1223-1232, XP002684427, ISSN: 0022-1554, DOI: 10.1177/002215540004800906
- JUNG S S ET AL: "Beta-amyloid precursor protein is detectable on monocytes and is increased in Alzheimer's disease", NEUROBIOLOGY OF AGING, TARRYTOWN, NY, US, vol. 20, no. 3, 1 May 1999 (1999-05-01), pages 249-257, XP002524773, ISSN: 0197-4580, DOI: 10.1016/S0197-4580(99)00051-2
- TAKEHIRO KIKO ET AL: "Amyloid [beta] Levels in Human Red Blood Cells", PLOS ONE, vol. 7, no. 11, 15 November 2012 (2012-11-15), page e49620, XP055294586, DOI: 10.1371/journal.pone.0049620
- NILSSON, K. ET AL.: 'Conjugated polyelectrolytes: conformation-sensitive optical probes for detection of amyloid fibril formation'.' BIOCHEMISTRY vol. 44, no. 10, 2005, pages 3718 - 3724, XP055284142
- ENGEL, V ET AL.: 'Sporadic inclusion-body myositis and its similarities to Alzheimer disease brain: recent approaches to diagnosis and pathogenesis, and relation to aging'.' SCANDINAVIAN JOURNAL OF RHEUMATOLOGY vol. 27, no. 6, 1998, pages 389 - 405, XP008181148
- SCHALL, U. ET AL.: 'Effekte von Interferonen auf die Phagozytosekapazität von mononukleären Phagozyten bei Alzheimer Erkrankung'.' DISS. SAARBRÜCKEN, UNIV., DISS., 2010 2008, XP055284145
- Adam S. Crystal ET AL: "A comparison of amyloid fibrillogenesis using the novel fluorescent compound K114", JOURNAL OF NEUROCHEMISTRY, vol. 86, no. 6, 14 August 2003 (2003-08-14), pages 1359-1368, XP055294189, NEW YORK, NY, US ISSN: 0022-3042, DOI: 10.1046/j.1471-4159.2003.01949.x

## Description

### RELATED APPLICATIONS

This application claims priority to US Patent Application No. 13/833,008 filed on March 15, 2013.

### FIELD

The disclosure relates to methods for analyzing blood to detect diseases associated with abnormal protein aggregation. In one embodiment, the disease associated with abnormal protein aggregation is Alzheimer's disease.

### BACKGROUND

Alzheimer's disease (AD) is the most common cause of dementia in elderly populations throughout the world with more than 35 million people affected, and is projected to rise to 115 million by 2050 if effective therapeutics are not developed (Barnes and Yaffe, 2011). This age-related neurodegenerative disorder is pathologically characterized by amyloid β (Aβ)-containing senile plaques, neurofibrillary tangles, and synapse loss in the brain (Selkoe 2001). Although it is clear that AD is a degenerative disorder, the role of the immune system is prominent (reviewed in Britschgi and Wyss-Coray 2007). In AD, toxic Aβ peptides aggregate into higher molecular weight assemblies and accumulate not only in the extracellular space, but also in the walls of blood vessels in the brain (de la Torre 2002; Deane and Zlokovic 2007), increasing their permeability (Nagababu et al 2009), and promoting transfer of T lymphocytes into brain (Farkas et al 2003). Macrophages/microglia ingest Aβ and are key players for Aβ clearance (Majumdar et al 2008; Mildner et al 2007; Simard et al 2006). Neutrophils also infiltrate into AD brain by virtue of blood brain barrier disruption (Stamatovic et al 2005).

Despite considerable effort, there is neither a cure for AD, nor even a clinical test to reliably establish the diagnosis with certainty; post-mortem examination of brain tissue is currently the only certain way to confirm the diagnosis of AD. In fact, current diagnostic criteria have "Probable AD" as the category with the highest certainty (McKhann et al 2011), reflecting the limitations in ante-mortem diagnosis. A simple and reliable test would be important for several reasons: therapeutic trials will be more reliable if enrolled subjects have a definitive diagnosis, allowing a more homogeneous population to be studied. Milder cases of dementia (mild cognitive impairment), where diagnostic criteria for AD are not yet met, could be correctly classified as early AD vs. other causes (e.g. vascular), allowing better prognostication and institution of proper treatment, once this becomes available.

From a population health perspective, given that biochemical changes in AD brain (e.g. amyloid deposition) begin years to perhaps decades before clinical symptoms, it may be possible to detect early pre-clinical disease and institute preventive measures when available. Thus, development of an inexpensive, non-invasive, rapid test for AD is of paramount importance as the developed world braces for this inevitable epidemic.

Much effort has gone into developing biomarkers to support an AD diagnosis Styren et al., 2000, The Journal of Histochemistry & Cytochemistry, 48(9), 1223-1232, focus on a fluorescent derivative of Congo red (X-34) as a marker of beta-sheet structures; Jung et al., 1999, Neurobiology of Aging, 249-257, rely on detection of beta-amyloid precursor protein on monocytes and Takehiro et al., 2012, PLOS One, 7(11), focus on the use of red blood cell Ab40 and Ab42 as biomarkers of AD. In the blood, focus has been on measuring Aβ levels in the plasma. This has not proved reliable, as levels of Aβ40, Aβ42, or ratios of the two, cannot reliably separate healthy controls from AD patients (Thambisety and Lovestone 2010). Other approaches include proteomics analysis of plasma, but this is expensive, complex, not amenable to high throughput assays, and remains experimental. CSF analysis of Aβ and (phosphorylated) tau levels has stronger predictive value (van Rossum et al 2012; Senanaron et al 2012), but compared to blood is invasive and unlikely to become routine outside of formal research trials. Imaging markers (MRI, fMRI, FDG-PET, amyloid-PET) (Jack 2012; Matsuda and Imabayashi 2012) are all more expensive, not universally available, and are either non-specific (MRI) or highly specialized and available in only a few centers (e.g, amyloid-PET). Therefore, a simple and inexpensive blood test to diagnose AD and AD-related mild cognitive impairment that will progress to AD, is highly desirable. An ability to perform detection from small samples of human blood would be a tremendous improvement over current methods, and pave the way for developing a simple, rapid high-throughput screening method. Such methods would also be applicable to other diseases characterized by abnormal protein aggregation.

### SUMMARY

The inventors describe a novel method for analyzing blood to detect diseases associated with abnormal protein aggregation. As set out in the Examples, the inventors have determined that it is possible to detect pathogenic protein aggregates associated with diseases including, but not limited to, Alzheimer's disease, Bovine Spongiform Encephalopathy (BSE) and traumatic brain injury in non-plasma blood elements such as circulating blood leukocytes and platelets. Furthermore, the use of spectral fluorescence techniques with fluorescent probes that bind pathogenic protein aggregates were shown to readily distinguish blood samples from healthy controls and blood samples from subjects with diseases associated with abnormal protein aggregation. The spectral analysis of non-plasma blood elements with fluorescent probes that bind pathogenic protein aggregates therefore allows for the detection of diseases typically associated with abnormal protein aggregation in the central nervous system. Using transgenic animal models, it was also shown that the methods described herein are able to identify subjects with Alzheimer's disease using blood samples early on during the progression of disease at the same time as pathology was observed in brain samples.

Accordingly, the disclosure relates to a method of detecting a disease associated with abnormal protein aggregation in a subject. In one embodiment, the method comprises (a) contacting non-plasma blood elements from the subject with a conformationally-sensitive probe that binds to pathogenic protein aggregates, wherein the non-pasma blood elements are selected from red blood cells, leukocytes, microvesicles and platelets (b) generating a fluorescence emission spectra or absorption spectra of the probe bound to the pathogenic protein aggregates, wherein the emission spectra or the absorption spectra of the conformationally-sensitive probe changes when the probe is bound to the pathogenic protein aggregate and (c) comparing the fluorescence emission spectra or absorption spectra of the probe contacted with the non-plasma blood elements to one or more reference fluorescence emission spectra or absorption spectra wherein the reference non-plasma blood elements are from a reference subject who has a disease associated with abnormal protein aggregation, and correspondence between the fluorescence emission spectrum or absorption spectra and the one or more reference fluorescence emission spectra or absorption spectra is indicative that the subject has a disease associated with abnormal protein aggregation.

The disclosure also relates to an *in vitro* method for detecting pathogenic protein aggregates in a blood sample from a subject. In a preferred embodiment, the non-plasma blood elements are leukocytes.

In one embodiment, leukocytes are isolated from the blood sample prior to contacting the leukocytes with a probe that binds to pathogenic protein aggregates.

In another embodiment, leukocytes of the buffy coat are contacted with a probe that binds to pathogenic protein aggregates.

In another embodiment, the probe is a fluorescent probe, optionally a conformationally-sensitive probe. Optionally, the probe is Congo Red, K114, X34, BSB, FSB, IMSB, Chrysamine-G, methoxy-X34, methoxy-X04, thioflavin-T, thioflavin-S, Pittsburgh compound B, thiazine red R, auramine-O, p-FTAA or a luminescent conjugated polythiophene (LCP) or luminescent conjugated oligothiophene (LCO) related to p-FTAA.

In one embodiment, the probe comprises an antibody that selectively binds to pathogenic protein aggregates. Optionally, the antibody is labeled with a detectable label such as a fluorescent label. In one embodiment, the probe is an anti β-amyloid antibody. In one embodiment, the probe is an anti-Tau or anti-hyperphosphorylated Tau antibody.

Detecting the probe bound to the pathogenic protein aggregates comprises detecting the fluorescence or absorbance of the probe bound to the pathogenic protein aggregates.

Detecting the fluorescence or absorbance of the probe comprises detecting the intensity of the fluorescence signal, generating a fluorescence emission spectrum and/or generating an absorption spectrum. In one embodiment, detecting the fluorescence of the probe bound to pathogenic protein aggregates comprises detecting the intensity of the fluorescence signal at a two of more wavelengths or the absorption of the probe bound to pathogenic protein aggregates at two or more wavelengths.

In another embodiment, generating a fluorescence emission spectrum comprises measurements at two or more wavelengths or generating an absorption spectrum comprising measurements at two or more wavelengths.

In another embodiment, at least one reference emission spectrum is a fluorescent emission spectrum from reference non-plasma blood elements from a reference subject who has a disease associated with abnormal protein aggregation, and correspondence between the fluorescence emission spectrum and the at least one reference fluorescence emission spectrum indicates that the subject has a disease associated with abnormal protein aggregation. Alternatively, at least one reference emission spectrum is a fluorescent emission spectrum from reference non-plasma blood elements from a reference subject who is a healthy control, such as a subject who does not have a disease associated with abnormal protein aggregation, and correspondence between the fluorescence emission spectrum and the at least one reference fluorescence emission spectrum indicates that the subject does not have a disease associated with abnormal protein aggregation. Optionally the reference emission spectra include non-neuronal disease controls.

In another embodiment, at least one reference absorption spectrum is an absorption spectrum from reference non-plasma blood elements from a reference subject who has a disease associated with abnormal protein aggregation, and correspondence between the absorption spectrum and the at least one reference absorption spectrum indicates that the subject has a disease associated with abnormal protein aggregation. Alternatively, at least one reference absorption spectrum is an absorption spectrum from reference non-plasma blood elements from a reference subject who is a healthy control, such as subject who does not have a disease associated with abnormal protein aggregation, and correspondence between the absorption spectrum and the at least one reference absorption spectrum indicates that the subject does not have a disease associated with abnormal protein aggregation.

A person skilled in the art will appreciate that in some embodiments, differences or similarities between the fluorescence spectra or absorbance spectra of subject non-plasma blood elements and the fluorescence or absorbance spectra of the reference non-plasma blood elements may indicate that the subject has or does not have a disease associated with abnormal protein aggregation depending on the disease state of the reference non-plasma blood elements. Similarly, differences or similarities between the fluorescence spectra or absorption spectra of subject non-plasma blood elements and the fluorescence spectra or absorption spectra of the reference non-plasma blood elements may indicate that the sample has pathogenic protein aggregates or does not have pathogenic protein aggregates depending on whether the reference non-plasma blood sample has pathogenic protein aggregates.

In another embodiment, the method comprises comparing a plurality of fluorescence emission spectra or absorption spectra with a plurality of reference spectra. For example, in one embodiment, two or more fluorescence emission spectra are generated using two or more different excitation wavelengths and compared to two or more reference spectra generated using the same or similar excitation wavelengths.

In another embodiment, the method further comprises: performing spectral unmixing to determine the weightings of individual basis spectra that contribute to the fluorescence emission spectrum or absorption spectrum; and
using the weightings to determine a probability that the subject has a disease associated with abnormal protein aggregation or using the weightings to determine a probability that a sample has a pathogenic protein aggregate.

In another embodiment, the individual basis spectra are determined from (a) samples of subjects known to have a disease associated with abnormal protein aggregation, (b) samples of healthy control subjects and/or (c) samples that have not been contacted with the probe. In one embodiment, the samples are non-plasma blood elements known to have or not have a particular pathogenic protein aggregate.

Different methods known in the art may be used to perform spectral unmixing in order to determine the weightings of individual basis spectra. For example, in one embodiment, spectral unmixing is performed using linear algebraic methods. In one embodiment, spectral unmixing is performed using non-linear algebraic methods. In another embodiment, the spectral unmixing is performed using the Levenberg-Marquardt algorithm.

Different methods known in the art may also be used to compare the fluorescence emission spectrum or absorption spectrum to one or more reference spectra in order to determine correspondence between the spectra, or to determine differences or similarities between the spectra. In one embodiment, suitable methods generate a quantitative measure of similarity or difference between spectra. In one embodiment, the methods described herein further comprises generating a statistical measure or probability score that a spectra or set of spectras are indicative of the presence of a disease associated with abnormal protein aggregation or the presence of pathogenic protein aggregates. For example, in one embodiment, machine learning, genetic algorithms, or principle component analysis may be used for comparing spectra or a set of spectra.

The disease associated with abnormal protein aggregation is selected from the group consisting of: Alzheimer's disease, mild cognitive impairment, cerebral amyloid angiopathy, brain trauma, traumatic brain injury, bovine spongiform encephalopathy or multiple sclerosis,

In another embodiment, the pathogenic protein aggregates comprise amyloid proteins, optionally β-amyloid, tau protein, hyperphosphorylated tau protein (pTau), prion protein and/or, αB-crystallin (CRYAB).

In another embodiment, the pathogenic protein aggregates comprise β-amyloids and the disease is Alzheimer's disease.

In another embodiment, the step of contacting leukocytes from the subject with the probe that binds to pathogenic protein aggregates is performed at an alkaline pH. For example, in one embodiment, the pH is above 9, above 10, or above 11. In one embodiment, the pH is optionally about 10.5. In another embodiment, the step of contacting leukocytes from the subject with the probe that binds to pathogenic protein aggregates is performed at an acidic pH, optionally a pH below 6, below 5 or below 4.

The methods described herein include generating a plurality of fluorescence measurements or a plurality of fluorescent spectra using a plurality of different excitation wavelengths. For example, in one embodiment the method comprises generating two or more fluorescence emission spectra at two or more excitation wavelengths. In one embodiment, the method comprises generating three, four, five, six, seven, eight, nine, ten or greater than ten fluorescence emission spectra at three, four, five, six, seven, eight, nine, ten or greater than ten excitation wavelengths, respectively. Without being limited by theory, varying the excitation wavelength and analyzing two or more fluorescence spectra is thought to increase the sensitivity and/or specificity of the methods described herein for the detection of pathogenic protein aggregates in blood.

In another embodiment, at least one of the excitation wavelengths is in the near-ultraviolet, optionally 200 nm to 400 nm. In one embodiment at least one of the excitation wavelengths is from 300 nm to 500 nm, optionally 375 nm or 445 nm. In another embodiment, the excitation wavelengths correspond to standard wavelengths of fixed semi-conductor laser diodes. In one embodiment, the excitation wavelengths include one or more wavelengths selected from 375 nm, 405 nm, 445 nm and 457 nm. In one embodiment, the at least one of excitation wavelengths is between 200 nm and 2000 nm.

In another embodiment, the fluorescent emission spectra or absorption spectra are generated by measuring the intensity of fluorescence or absorption at a plurality of wavelengths. In one embodiment, the spectra are generated by taking measurements from 400 nm to 700 nm or from 200nm to 2000nm.

In another embodiment, the fluorescence or absorption of the probe bound to the pathogenic protein aggregates is normalized to cell count. In one embodiment, the fluorescence or absorption of the probe bound to the pathogenic protein aggregates is normalized to cell count in the sample being measured for fluorescence or absorption, such as within a light path or within an imaging field. Different methods known in the art for determining cell count include quantifying or estimating the number of cells in a sample such as by use of a fluorophore such as DRAQ5 to stain cell nuclei.

In another embodiment, detecting the probe bound to the pathogenic protein aggregates comprises measuring the fluorescence or absorbance of the probe bound to the pathogenic protein aggregates in a sample of non-plasma blood elements in a cuvette or microtiter plate.

In another embodiment, detecting the probe bound to the pathogenic protein aggregates comprises measuring the fluorescence or absorbance of the probe bound to the pathogenic protein aggregates in all or part of a 2-dimensional field comprising a sample of non-plasma blood elements.

In one embodiment the non-plasma blood elements are leukocytes or platelets.

In another embodiment, the probe is Congo Red, K114, X34, BSB, FSB, IMSB, Chrysamine-G, methoxy-X34, methoxy-X04, thioflavin-T, thioflavin-S, Pittsburgh compound B, thiazine red R, auramine-O or p-FTAA, a luminescent conjugated polythiophene (LCP) or luminescent conjugated oligothiophene (LCO) related to p-FTAA.

In another embodiment, detecting the probe bound to the pathogenic protein aggregates comprises detecting the fluorescence or absorbance of the probe bound to the pathogenic protein aggregates.

In another embodiment, detecting the fluorescence or absorbance comprises detecting the intensity of the fluorescence signal, generating a fluorescence emission spectrum or generating an absorption spectrum as described herein.

In another embodiment, the pathogenic protein aggregates comprise amyloid proteins, optionally β-amyloid, and/or tau protein, hyperphosphorylated tau protein (pTau), prion protein, αB-crystallin (CRYAB).

In another embodiment, the pathogenic protein aggregates comprise β-amyloids and the disease is Alzheimer's disease.

Other features and advantages of the disclosure will become apparent from the following detailed description. It should be understood, however, that the description and the specific examples while indicating preferred embodiments are given by way of illustration only.

### DRAWINGS

Embodiments are described below in relation to the drawings in which:

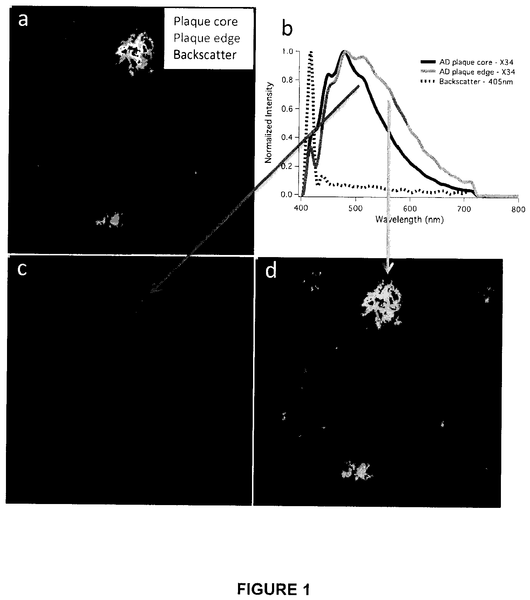

Figure 1 depicts spectral fluorescence images of X-34, a fluorescent amyloid probe (Styren et al. 2000), upon binding to senile plaques in formalin-fixed sections from 5xFAD, AD transgenic mouse brains. 32 channels of spectrally resolved data are collected for each pixel. **a**) Unmixed image showing typical morphology of amyloid plaques and two states of plaque "maturity". **b**) Measured emission spectra from plaque and laser backscatter used in the mathematical unmixing operation. The closely spaced emissions of the core and edge of a large amyloid plaque in the X-34 image were well separated as shown in **c** and **d.** These results illustrate the ability not only to detect amyloid plaques in brain, but to be able to distinguish various aggregation states within a single plaque.

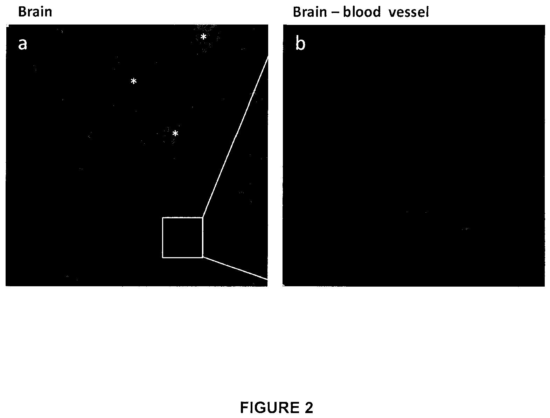

Figure 2 shows anti-Aβ (6E10) immunofluorescence in formalin-fixed sections from 5xFAD mouse brain. a) Typical morphology of senile plaques which is 6E10 immuno-positive (asterisks). 6E10-positive blood cells are also observed around a blood vessel (Square box). b) High magnification images of the square box from a, showing that the leukocytes contain material that is Aβ.

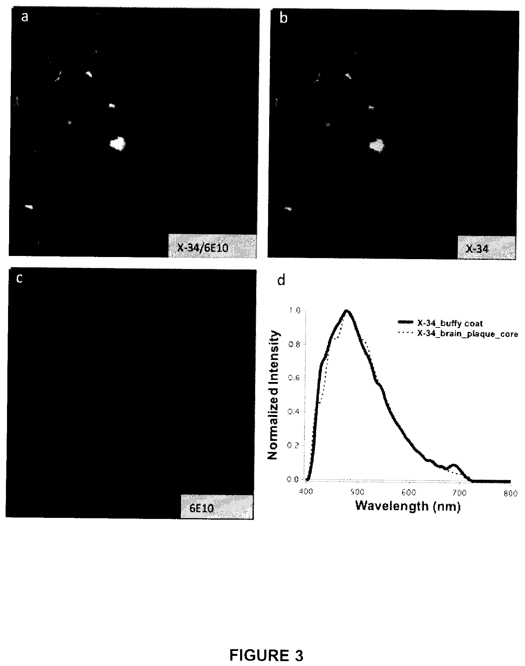

Figure 3 shows double labeling of X-34 with 6E10 immunofluorescence in mouse buffy coat samples. a-c) Unmixed X-34 image with 6E10 immunofluorescence in 5xFAD mouse buffy coat from circulating blood. X-34-positive cells are clearly co-localized with 6E10 immunofluorescent signals, d) Emission spectra of X-34-positive 5xFAD mouse buffy coat leukocytes. Measured emission spectra of X-34-positive leukocytes from circulating blood are virtually identical to those from senile plaque in 5xFAD mouse brain (dotted line), demonstrating that the leukocytes contain material that is very similar to that found in plaques.

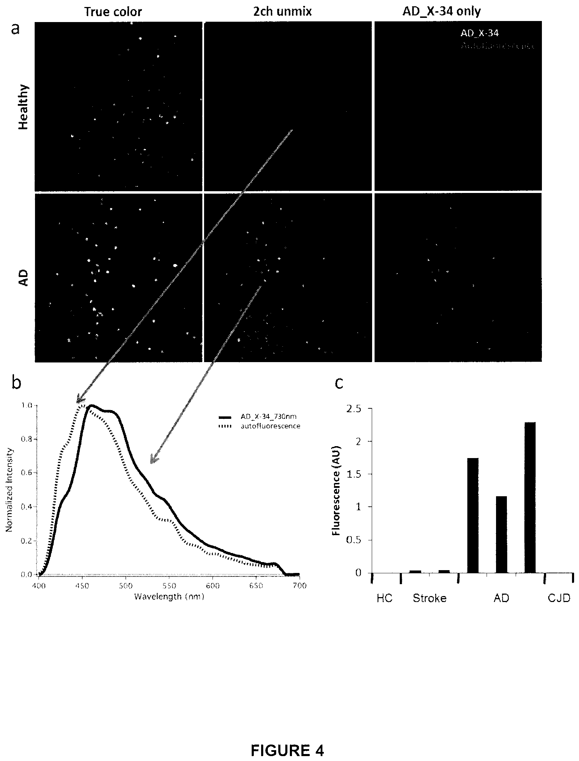

Figure 4 shows 2-photon spectral fluorescence images of X-34 upon binding to amyloid-like material in buffy coat from healthy control (HC) and AD patients, a) Truecolor (left column), unmixed autofluorescence (grey) and AD-specific X-34 (white) channels (middle & right columns). AD-specific X-34 signal was detected only from AD buffy coat. b) Emission spectra of AD-specific X-34 signals and autofluorescence. c) A bar graph showing strong AD-specific signal only in AD leukocytes. CJD refers to a patient with Creutzfeldt-Jakob disease.

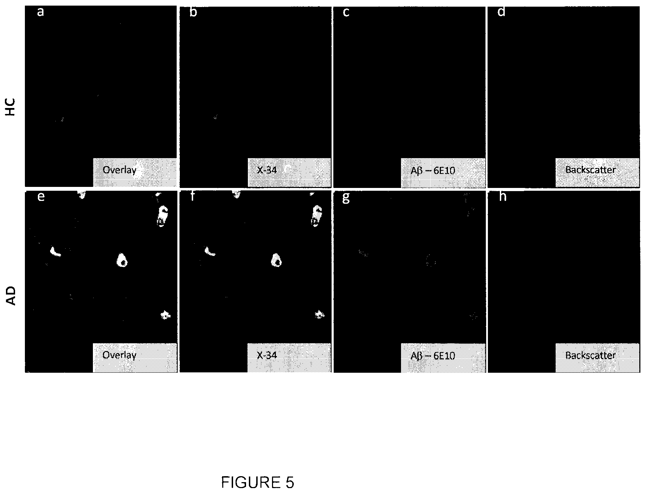

Figure 5 shows double-labeling using the amyloid probes X-34, with anti-Aβ antibody (6E10) in buffy coat from healthy control (HC) and AD patients. AD-specific X-34 signal (see Fig.4 for details) is strong only in AD leukocytes (f), which co-localizes with strong Aβ immunolabeling (g). Only a fraction of AD leukocytes were positive however. In contrast, only very weak background signal is seen in the healthy control buffy coat (b, c). Laser backscatter conveniently identifies each cell (d, h), and this signal is perfectly separated from the other emissions using the spectral techniques described herein.

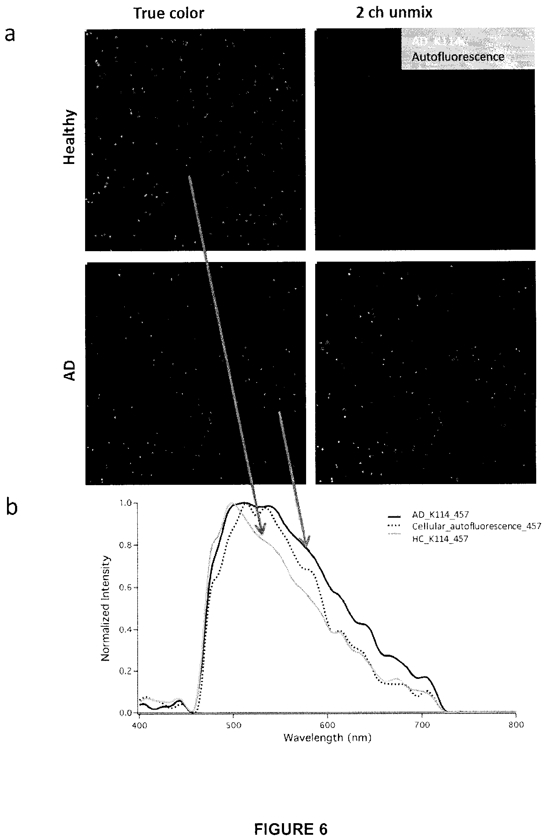

Figure 6 shows spectral fluorescence images of K114, another fluorescent amyloid probe (Crystal 2003), upon binding to amyloid-like material in buffy coat from healthy control (HC) and AD patients. a) Truecolor (left column), unmixed autofluorescence and AD-specific K114 channels (right column). b) Emission spectra of HC-, AD-specific K114 signals and autofluorescence.

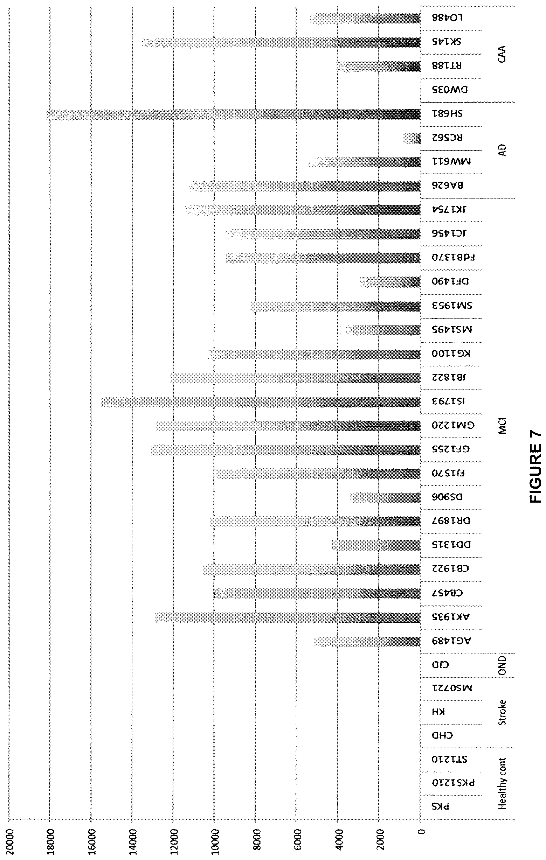

Figure 7 is simplified composite index that reflects the probability that a subject has either AD or Mild Cognitive Impairment (MCI) ("ADR index" ie "AD related index"). Leukocyte cells obtained from patients were labeled with the amyloid probe K114 and spectrally analyzed as above.

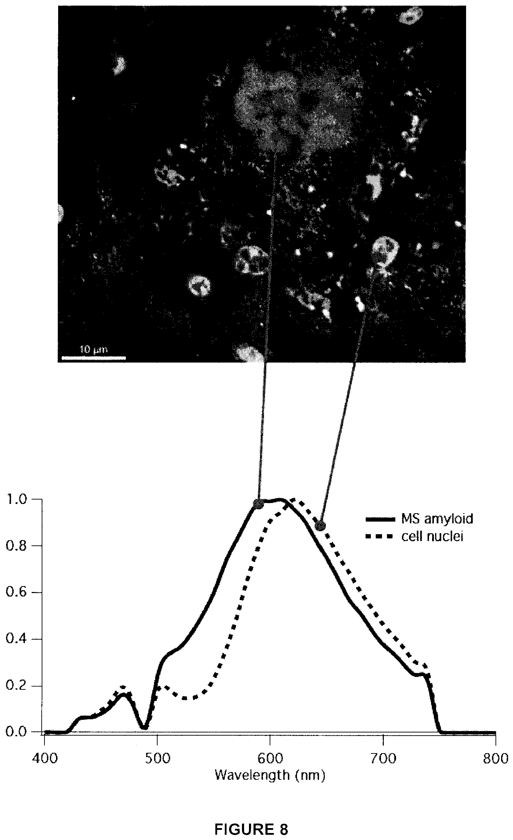

Figure 8 shows human brain from a multiple sclerosis patient stained with Congo Red. These data suggest that chronic MS plaques have deposits of amyloid, which should be detectable in blood leukocytes, in an analogous manner to AD.

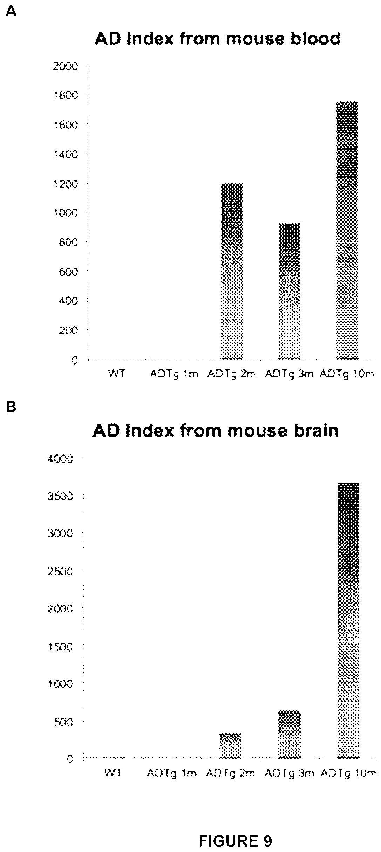

Figure 9 shows the AD index obtained by spectral analysis of blood (Figure 9A) and brain (Figure 9B) samples from a transgenic mouse model of Alzheimer's disease (5xFAD) at 1 month, 2 months, 3 months and 10 months as well as samples from a wild-type (WT) control using the probe K114 as described in Example 6.

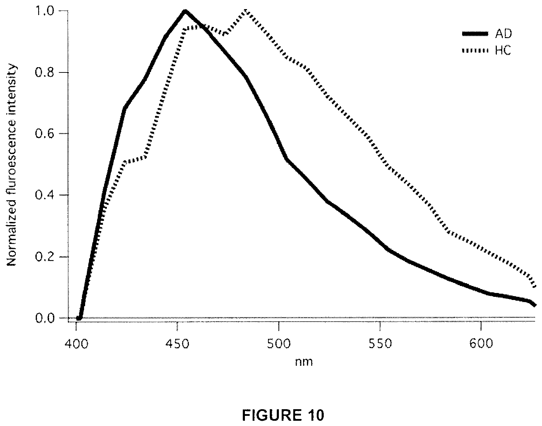

Figure 10 shows spectra obtained from analyzing blood from a subject diagnosed with Alzheimer's disease (AD) compared to a healthy control (HC) as described in Example 7.

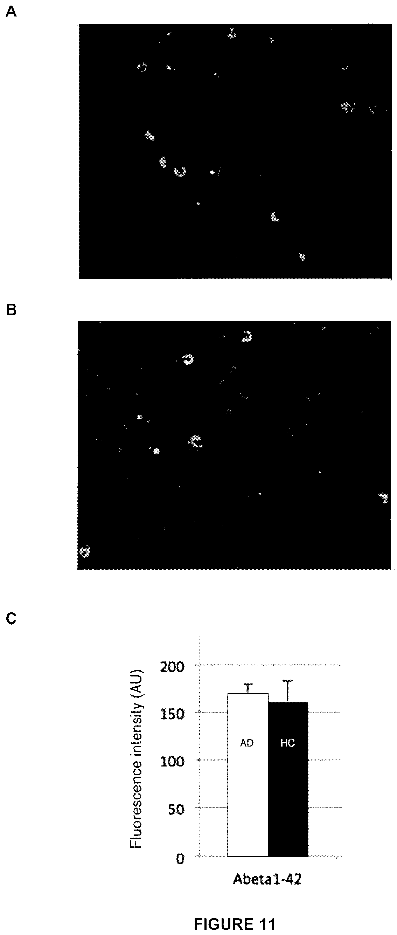

Figure 11 shows staining of leukocytes obtained from a healthy control (Figure 11A) and a subject with Alzheimer's disease (AD) (Figure 11B) with an antibody for Aβ₁₋₄₂ and a probe for pathogenic protein aggregates (K-114). Both healthy control and AD subjects exhibit Aβ₁₋₄₂ staining, however Figure 11B shows a much higher level of staining for K-114 indicating the presence of pathogenic protein aggregates. Leukocytes typically exhibit small bright inclusions as well as more diffuse cytoplasmic staining, both of which are positive for amyloid probe labeling. The inclusions occur in both healthy control and AD blood, but are more numerous in AD samples and display unique spectral signatures; cytoplasmic label on the other hand is far stronger in AD samples and virtually undetectable in healthy control leukocytes. Aβ₁₋₄₂ immunolabels a subset of leukocytes, and labels both granules and diffuse cytosol. Figure 11C shows that the overall intensity of the Aβ₁₋₄₂ immunolabel is similar in AD vs. healthy control (HC).

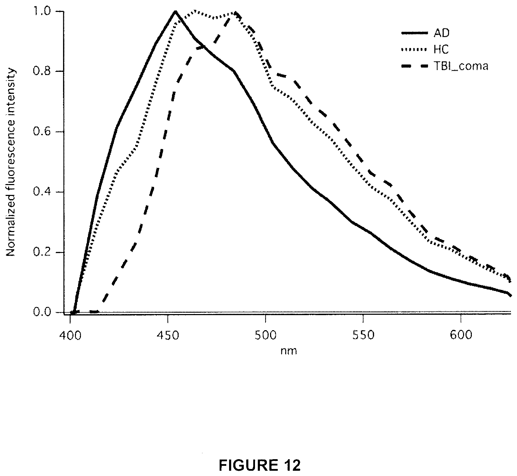

Figure 12 shows spectra obtained by analyzing leukocytes from a subject still in a coma 5 days after a traumatic brain injury (TBI) with a probe that binds to pathogenic protein aggregates (K-114) compared to spectra obtained from a subject with Alzheimer's disease (AD) and a healthy control (HC).

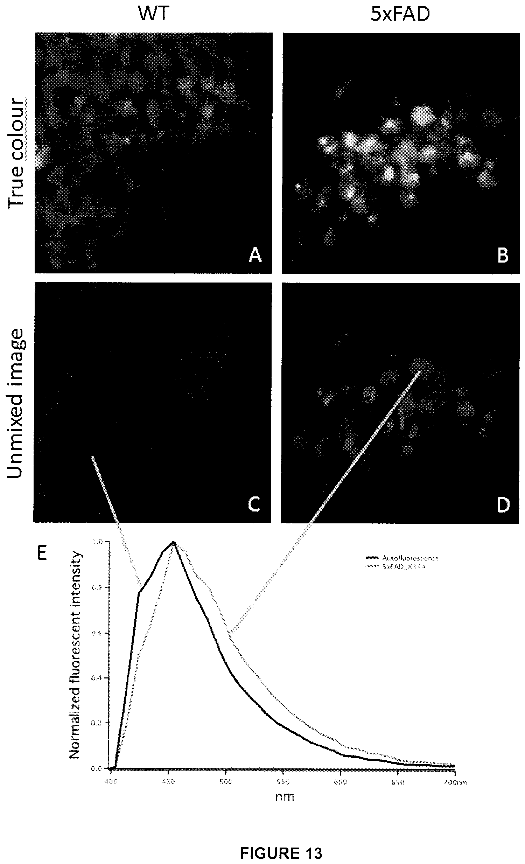

Figure 13 shows staining of platelets obtained from wild type control and 5xFAD mice with K-114. Figures 13A and 13B show K114 true colour images, while Figures 13C and 13D show the unmixed K114 image. Figure 13E shows the emission spectra of Aβ-specific K114 signals and autofluorescence. 5xFAD platelets show higher Aβ-specific K114 signals compared to wild type control platelets (Figures 13B and 13D).

### DETAILED DESCRIPTION

The inventors have discovered that the spectral signatures of amyloid probe fluorescence in non-plasma blood elements from AD subjects are highly similar to those from human and mouse brain senile plaques, thereby demonstrating that non-plasma blood elements contain material that is similar to the material found in cerebral plaques. Without being bound by theory, it is postulated that non-plasma blood elements such as leukocytes, platelets and/or red blood cells exposed to pathogenic protein aggregates accumulate these aggregates. The present methods link the presence of pathogenic protein aggregates in circulating non-plasma blood elements to the diagnosis of diseases associated with abnormal protein aggregation. Also described are methods for detecting and/or identifying pathogenic protein aggregates in non-plasma blood elements.

Accordingly, an aspect of the disclosure provides a method of detecting a disease associated with abnormal protein aggregation in a subject, wherein the presence of pathogenic protein aggregates in the non-plasma blood elements of the subject is indicative that the subject has a disease associated with abnormal protein aggregation. Another aspect of the disclosure provides a method for detecting pathogenic protein aggregates in a blood sample from a subject by contacting the sample with a probe that binds pathogenic protein aggregates and optionally identifying the type or composition of pathogenic protein aggregate in the sample by spectral analysis.

As used herein, the term "disease associated with abnormal protein aggregation" refers to any disease that is associated with and/or characterized by protein misfolding and aggregation. In such diseases, proteins may be misfolded and aggregated from their soluble functional state into highly ordered fibrillar assemblies with high β-sheet content ("amyloid peptides" or "amyloids"). While "diseases associated with abnormal protein aggregation" may be otherwise unrelated, they have in common binding to amyloid probes such as Congo Red. Diseases associated with abnormal protein aggregation include, but are not limited to, Alzheimer's disease, Parkinson's disease, Huntington's disease, mild cognitive impairment, cerebral amyloid angiopathy, brain trauma, frontotemporal dementia, Pick's disease, multiple sclerosis, prion disorders, Down's syndrome and Amyotrophic Lateral Sclerosis (ALS). Other disease associated with abnormal protein aggregation include neuromuscular disorders such as myopathies (e.g. desminopathies, alpha-B crystallinopathies, selenoproteinopathies, hereditary inclusion body myopathies, actinopathies, myosinopathies) (Vicart et al 1998) and neuropathies such as familial amyloidosis neuropathy (Sharma and Goebel 2005). Diseases associated with abnormal protein aggregation also include neurological disorders and/or neurodegenerative disorders. Diseases associated with abnormal protein aggregation are not limited to the specific diseases recited herein; any disease that includes misfolded protein aggregates as part of its pathology may be detected using the methods described herein.

Different proteins may be misfolded and aggregated in different diseases. For example, Alzheimer's disease is characterized by β-amyloid containing plaques that accumulate in brain tissue. In Parkinson's disease, α-synuclein (a-Syn) neuronal inclusions are seen. Huntington's disease is associated with aggregations of the huntingtin protein. The infectious agent responsible for prion disorders such as transmissible spongiform encephalopathy, bovine spongiform encephalopathy and Creutzfeldt-Jakob disease (CJD) is a misfolded and aggregated version of prion protein. Further, the present inventors have evidence that amyloid deposits are found in the brains of multiple sclerosis patients.

As used herein, the term "pathogenic protein aggregates" refers to toxic misfolded and aggregated versions of proteins and polypeptides present in the body. As used herein, the term "protein aggregates" refers to misfolded peptide or protein oligomers, including peptide dimers and oligomers larger than dimers. Examples of "pathogenic protein aggregates" include amyloids. "Amyloids" is a general term used to describe insoluble fibrous protein aggregates. Pathogenic protein aggregates can also comprise soluble aggregates such as β-amyloid oligomers. Pathogenic protein aggregates can be made up of a single protein or multiple different proteins. Pathogenic protein aggregates can also include lipid and nucleic acid components. Examples of proteins that can be found in pathogenic protein aggregates include, but are not limited to, β-amyloid, α-synuclein, huntingtin, tau protein, hyperphosphorylated tau protein (pTau), prion protein, αB-crystallin (CRYAB), desmin, selenoproteins, actin and myosin as well as superoxide dismutase (SOD).

As used herein the phrase "method of detecting a disease associated with abnormal protein aggregation in a subject" refers to a method or process of determining if a subject has a disease associated with abnormal protein aggregation. The phrase "method of detecting a disease associated with abnormal protein aggregation in a subject" also refers to a method of determining if a subject has an increased risk of developing a disease associated with abnormal protein aggregation. The terms "risk" and "increased risk" as used herein refer to a subject having a predisposition to developing a disease e.g increased risk compared to the average risk of a population. The phrase "detecting a disease" also refers to detecting a disease in a pre-symptomatic patient. "Detecting a disease" also includes detecting the stage, severity, subtype or the progression of a disease.

In one embodiment, the methods described herein include obtaining a blood sample from a subject. Methods of obtaining blood samples are well known in the art. In one embodiment, the sample comprises blood, whole blood or a fraction thereof. In another embodiment, the blood sample comprises buffy coat. In a further embodiment, the blood sample comprises leukocytes. As used herein "non-plasma blood elements" refers to the constituents of blood other than plasma such as red blood cells, leukocytes, microvesicles and platelets.

Buffy coat is a fraction of an anticoagulated blood sample following density gradient centrifugation of the blood. The buffy coat contains most of the leukocytes and platelets of the blood. The buffy coat may be isolated from a blood sample using any technique known in the art. Optionally, blood samples may be centrifuged and the buffy coat (or leukocyte band) may be removed. In one embodiment, if a red blood cell free sample is desired, any contaminating red blood cells are lysed.

As used here, the term "leukocyte" or "white blood cell" refers to a cell of the immune system. Examples of leukocytes include granulocytes such as neutrophils, eosinophils and basophils, lymphocytes, monocytes and macrophages (derivatives of monocytes). In one embodiment, pathogenic protein aggregates are identified in phagocytic blood leukocytes including, but not limited to, monocytes and/or macrophages. Without being bound by theory, it is believed that all major types of leukocytes can potentially accumulate pathogenic Aβ or other misfolded proteins/peptides, then carry these to circulating blood. Phagocytosis by macrophages and neutrophils is a key line of defense, clearing pathogens and waste products. Further, while lymphocytes are not phagocytic, intercellular protein transfer has been observed between these immune cells (termed trogocytosis) (reviewed in Caumartin et al 2006).

As used herein, the term "isolating leukocytes" refers to obtaining a sample comprising leukocytes, consisting of leukocytes or consisting essentially of leukocytes. In one embodiment, isolating leukocytes refers to obtaining a sample that is free of, or substantially free of, non-leukocyte cells. The term "isolating leukocytes" optionally refers to isolating the buffy coat layer of a blood sample. The methods described herein are performed on a sample of leukocytes, such as sample comprising leukocytes, consisting of leukocytes or consisting essentially of leukocytes. In another embodiment, the methods described herein are performed on a buffy coat sample.

In another embodiment, a particular type of leukocyte cell is isolated and the methods described herein are performed on that particular sub-type. Examples of leukocyte cell types include neutrophils, eosinophils and basophils, lymphocytes, NK cells, monocytes and macrophages.

As shown in Figure 13, blood platelets associated with pathogenic protein aggregates show distinctive spectra when stained with K114 and can be differentiated from platelets derived from healthy controls. Other non-plasma blood elements such as red blood cells and microvesicles may also accumulate pathogenic Aβ or other misfolded proteins/peptides, then carry these in circulating blood.

The methods described herein include the identification of pathogenic protein aggregates in the non-plasma blood elements of a subject. The presence of pathogenic protein aggregates in non-plasma blood elements can be detected using a number of methods. In one embodiment, pathogenic protein aggregates are detected in non-plasma blood elements using probes that specifically bind to and/or interact with the pathogenic protein aggregates.

The methods described herein also include the identification of pathogenic protein aggregates associated with the non-plasma blood elements of a subject. The presence of pathogenic protein aggregates associated with non-plasma blood elements can be detected using a number of methods. As used herein, the term "associated with" non-plasma blood elements is used to refer to pathogenic protein aggregates adsorbed or absorbed to the surface of non-plasma blood elements, absorbed into the interior of non-plasma blood elements, or in separated granules outside of the non-plasma blood elements. In one embodiment, the term "associated with" non-plasma blood elements refers to pathogenic protein aggregates adsorbed or absorbed to the leukocyte cell surface, absorbed into the leukocyte interior, or in separated granules outside of the leukocyte cells.

In one embodiment, pathogenic protein aggregates are detected in non-plasma blood elements such as leukocytes using probes that specifically bind to and/or interact with the pathogenic protein aggregates. As used herein, the term "probe that binds to pathogenic protein aggregates" includes both direct and indirect binding to the aggregates.

As used herein, the term "probe" refers to any detectable agent that binds, directly or indirectly, to a pathogenic protein aggregate. In one embodiment, the probe is a fluorescent probe or a luminescent probe. A "fluorescent probe" is a probe with the ability to emit light of a certain wavelength when activated by light of another wavelength.

"Conformationally-sensitive" indicates that the absorption and/or emission spectra, as well as emission intensity, of the fluorescent and/or luminescent probe change as a function of the conformation of the target peptide, protein, lipid, nucleic acid, or macromolecular assembly thereof, thereby reporting the conformational states of such assemblies or aggregates. Probes can be engineered to exhibit maximal spectral changes as a function of the conformational state of said macromolecular aggregates to maximize sensitivity and specificity of detection of the pathogenic aggregates.

In another embodiment, the probe specifically binds to amyloid fibrils. Specific examples of conformationally-sensitive fluorescent probes useful for detecting amyloid peptides include, but are not limited to, Congo Red, Congo Red derivatives and bis-styrylbenzenes (e.g. K114, X34, BSB, FSB, IMSB, Chrysamine-G, methoxy-X34, methoxy-X04), thioflavin analogs (thioflavin-T, thioflavin-S, Pittsburgh compound B), thiazine red R, auramine-O, pentameric formyl thiophene acetic acid (p-FTAA) and related luminescent conjugated polythiophenes (LCPs) or luminescent conjugated oligothiophenes (LCOs). As used herein, the term "contacting a cell with a probe" or "labeling a cell with a probe" refers to any means by which a cell is exposed to a probe such that the probe is able to bind pathogenic protein aggregates in the cell (i.e., intracellular protein aggregates) or protein aggregates associated with the cell. In one embodiment, cells are permeabilized prior to contacting with a probe or labeling with a probe. Methods of labeling non-plasma blood elements such as cells with fluorescent probes are well known in the art. For example, leukocytes are optionally fixed and dried and then stained with a fluorescent probe. The pH, time, concentration and vehicle for the probe may be specific for the probe. In one embodiment, the inventors have determined the probe is preferably contacted with the non-plasma blood elements at an alkaline pH, such as a pH above 10 or about 10.5. This results in improved sensitivity and/or specificity for detecting pathogenic protein aggregates using the spectroscopic methods as described herein.

Once the non-plasma blood elements are labeled, various methods may be used to detect binding of the fluorescent probe to the protein aggregates. In one embodiment, detecting the probe bound to the pathogenic protein aggregates comprises detecting the fluorescence of the probe bound to the pathogenic protein aggregates. Detecting the fluorescence of the probe may be accomplished by any method known in the art.

The "absorbance" of the probe is the amount of light the probe absorbs at any particular wavelength. Detecting the absorbance of the probe may be accomplished by any method known in the art. In another embodiment, detecting the absorbance of the probe comprises generating absorption spectra.

In one embodiment, probe fluorescence or absorbance is spectrally resolved using a spectrometer. The spectrometer is optionally an imaging or a non-imaging spectrometer. In another embodiment, a wide-field spectral camera is used to detect probe emission.

In one embodiment, fluorescence or absorbance is detected using fluorescence spectroscopy. In one embodiment, spectral 2-photon imaging is used. For example, spectral 2-photon imaging may be performed with a conventional narrow-band ultrafast laser (e.g. Chameleon, Coherent Inc.) or ultrabroadband femtosecond laser (Octavius, Thorlabs) and a spectral laser-scanning microscope such as the Nikon A1RMP. In another embodiment, spectral multiphoton or spectral 1-photon imaging is used. One or several excitation wavelengths may be used to excite the fluorescent probes, including the entire UV, near-UV, visible and near-infrared. In one embodiment, the excitation wavelength is between about 200 nm and about 2000 nm, and optionally between about 400 nm and 700nm. Images may be analyzed using various programs such as the program ImageTrak (http://www.ucalgary.ca/styslab/imagetrak).

In another embodiment, fluorescence or absorbance is detected using a non-imaging spectrometer such as from a cuvette, small sample holder or a multi-well plate. Here, the labeled cells are suspended, excited by one or more laser lines, and the emission is collected with a (non-imaging) spectrometer.

In yet another embodiment, fluorescence or absorbance is detected using single-cell spectral analysis as in a FACS analyzer. In another embodiment, a microplate reader is used.

In one embodiment, detecting the fluorescence of the probe comprises detecting the intensity of the fluorescence signal from the probe. In another embodiment, detecting the fluorescence of the probe comprises detecting the intensity of the protein-aggregate specific fluorescence from the probe. The term "protein aggregate specific fluorescence" refers to fluorescence from a probe that binds to protein aggregates that is specific for the pathogenic protein aggregates and is not attributable to background fluorescence, autofluorescence or normal state protein.

In another embodiment, detecting the absorbance of the probe comprises detecting the degree, intensity, or quantity of the absorption by the probe. In another embodiment, detecting the absorption of the probe comprises detecting the intensity, degree or quantity of the protein-aggregate specific absorbance by the probe. The term "protein aggregate specific absorbance" refers to absorbance by a probe that binds to protein aggregates that is specific for the pathogenic protein aggregates and is not attributable to background absorption, autoabsorption or normal state protein.

Preferably, the reference probe and the probe used to contact the subject non-plasma blood elements is the same probe.

In one embodiment, reference non-plasma blood elements, or control non-plasma blood elements, are non-plasma blood elements derived from a reference subject who has a disease associated with abnormal protein aggregation. In another embodiment, reference non-plasma blood elements, or control non-plasma blood elements, are non-plasma blood elements derived from a reference subject who does not have a disease associated with abnormal protein aggregation. The reference non-plasma blood elements are optionally tested at the same time as the subject non-plasma blood elements. In another embodiment, the reference non-plasma blood elements are tested at different time from the subject non-plasma blood elements. Optionally, the reference spectra are pre-determined or standardized control spectra representative of non-plasma blood elements with particular pathogenic protein aggregates or non-plasma blood elements from subjects with a disease associated with pathogenic protein aggregates. As used herein, the term "subject non-plasma blood elements" refers to non-plasma blood elements derived from a test subject. In a preferred embodiment, the non-plasma blood elements are leukocytes.

Correspondence, or similarity, between the fluorescence or absorbance of subject non-plasma blood elements and the fluorescence or absorbance of reference non-plasma blood elements from a reference subject who has a disease associated with abnormal protein aggregation indicates that the subject has a disease associated with abnormal protein aggregation. Differences between the fluorescence or absorbance of subject non-plasma blood elements and the fluorescence or absorbance of the reference non-plasma blood elements from a reference subject who has a disease associated with abnormal protein aggregation indicates that the subject does not have a disease associated with abnormal protein aggregation.

Likewise, correspondence, or similarity, between the fluorescence or absorbance of subject non-plasma blood elements and the fluorescence or absorbance of the reference non-plasma blood elements from a reference subject who does not have a disease associated with abnormal protein aggregation indicates that the subject does not have a disease associated with abnormal protein aggregation.

Correspondence, or similarity, between the fluorescence or absorbance of subject non-plasma blood elements and the fluorescence or absorbance of reference non-plasma blood elements is optionally determined by the amount of fluorescence, the intensity of the fluorescence or the nature of the fluorescence or the degree of absorbance. In another embodiment, correspondence, or similarity, between the fluorescence or absorbance of subject non-plasma blood elements and the fluorescence or absorbance of reference non-plasma blood elements is determined by analyzing fluorescence emission spectra or absorption spectra, for example by identifying similar fluorescence emission spectrum or absorption spectrum patterns or similar peaks and troughs in the fluorescence emission spectra or absorption spectra.

In another embodiment, the identification of an increase in fluorescence or absorbance, optionally a statistically significant increase in fluorescence or absorbance, of subject non-plasma blood elements compared to reference non-plasma blood elements from a patient who does not have a disease associated with abnormal protein aggregation indicates that the test subject has a disease associated with abnormal protein aggregation.

In another embodiment, the identification of a similar amount of fluorescence or absorbance from subject non-plasma blood elements compared to reference non-plasma blood elements from a patient who has a disease associated with abnormal protein aggregation indicates that the test subject has a disease associated with abnormal protein aggregation. In one embodiment, a "similar amount" of fluorescence or absorbance refers to no statistically significant difference in fluorescence or absorbance.

In one embodiment, an increase or decrease in fluorescence can be determined visually by looking at spectral fluorescence images. Increased fluorescence or absorbance can also be quantified. In one embodiment, at least a 10%, 25%, 50%, 75% or 100% increase in fluorescence or absorbance from subject non-plasma blood elements compared to reference non-plasma blood elements from a patient who does not have a disease associated with abnormal protein aggregation indicates that the test subject has a disease associated with abnormal protein aggregation. In another embodiment, at least a 10%, 25%, 50%, 75% or 100% decrease in fluorescence or absorbance from subject non-plasma blood elements compared to reference non-plasma blood elements from a patient who has a disease associated with abnormal protein aggregation indicates that the test subject does not have a disease associated with abnormal protein aggregation.

The use of fluorescence or absorbance spectroscopy also allows spectral signatures or fluorescence emission spectra or absorption spectra to be generated from non-plasma blood elements labeled with a fluorescent probe. A fluorescence emission spectrum or absorption spectrum plots the normalized intensity of the fluorescent or absorbance signal from the probe against the wavelength for a given sample. The fluorescence emission spectrum or absorption spectrum thereby provides a specific fluorescent or absorbance signature for a given sample.

In one embodiment, the present methods include comparing the fluorescence emission spectrum or absorption spectrum of subject non-plasma blood elements to the fluorescence emission spectrum or absorption spectrum of one or more reference non-plasma blood elements.

Correspondence, or similarity, between the fluorescence emission spectrum or absorption spectrum of subject non-plasma blood elements and a reference fluorescence emission spectrum or absorption spectrum of non-plasma blood elements from a reference subject who has a disease associated with abnormal protein aggregation indicates that the subject has a disease associated with abnormal protein aggregation. Correspondence, or similarity, between the fluorescence emission spectrum or absorption spectrum of subject non-plasma blood elements and a reference fluorescence emission spectrum or absorption spectrum of non-plasma blood elements from a reference subject who does not have a disease associated with abnormal protein aggregation indicates that the subject does not have a disease associated with abnormal protein aggregation. As used herein, correspondence, or similarity, between fluorescence emission spectra or absorption spectra is determined, for example by identifying similar fluorescence emission spectrum or absorption spectrum patterns or similar peaks or troughs in the fluorescence emission spectra or absorption spectrum. In one embodiment, fluorescence emission spectra or absorption spectrum correspond if at least 60, 70, 80, 90 or 95% of the spectral signatures overlap or substantially overlap.

It has been shown that the spectral signatures of the leukocyte amyloid probe fluorescence from AD subjects are similar to those from human and mouse brain senile plaques. Thus, in another embodiment, a reference fluorescence emission spectrum or absorption spectrum is the fluorescence emission spectrum or absorption spectrum from a labeled plaque sample derived from a subject who has a disease associated with abnormal protein aggregation. Optionally, the plaque sample is from an AD-related plaque from a subject with AD. If the fluorescence emission spectrum or absorption spectrum of the test sample corresponds to, or is similar to, the reference emission spectrum or absorption spectrum from the plaque sample, then the test sample comprises pathogenic protein aggregates and/or the test subject has a disease associated with abnormal protein aggregation.

In another embodiment, fluorescence emission spectra or absorption spectra are analyzed to generate an index indicating disease probability. Here, the spectra are analyzed as described below to extract the various components and an index indicating disease probability is assigned. A score consisting of specific emission spectra or absorption spectra, above a particular threshold, indicates that the test cell sample comprises pathogenic protein aggregates and/or the test subject has a disease associated with abnormal protein aggregation.

In one embodiment, a numerical score based on fluorescence emissions or absorbance is calculated. The numerical score reflects the probability that a test cell sample originated from a patient with a disease associated with abnormal protein aggregation. Here, spectral 1-photon confocal, 2-photon or multi-photon images are acquired and fluorescence and/or absorbance signals from non-plasma blood elements are processed using algorithms (for example, algorithms based on the Levenberg-Marquardt damped least-squares non-linear curve fitting algorithm, or linear algebraic unmixing/decomposition algorithms) to separate disease-specific signals from background and auto-fluorescence emission. Quantitative fluorescence/absorbance data are extracted thereby allowing the reliable identification of disease versus non-disease samples. In one embodiment, the mathematical method generates numerical scores associated with samples that originate from a subject who has a disease associated with abnormal protein aggregation and numerical scores associated with samples that originate from a subject who does not have a disease associated with abnormal protein aggregation. In one embodiment, the difference between the disease-associated and non-disease associated numerical scores is large enough to allow the classification of test samples as disease-associated or non-disease associated.

In one embodiment, the fluorescence emission spectrum or absorption spectrum acquired from non-plasma blood elements contacted with the fluorescent probes described herein is a linear composite of individual basis spectra generated by different species of misfolded proteins, cellular autofluorescence and background signal. These basis spectra contribute different intensities to the overall composite, which is a linear sum of the contributing spectra. The unmixing operation is designed to calculate in a quantitative manner, the relative contributions of each basis spectrum to the overall composite. Once basis spectra are determined from standard samples (for example, known healthy controls, known patients with a disease associated with abnormal protein aggregation such as AD, cellular autofluorescence from unstained samples), the composite spectrum is unmixed using an algorithm such as the Levenberg-Marquardt algorithm to determine the weightings of each basis spectrum that contributed to the composite. These weighting coefficients are then used to calculate the various indexes that indicate the probability that any one sample originated from a healthy or diseased subject.

Accordingly, in one embodiment, the methods comprise:
a. generating a fluorescence emission spectrum or absorption spectrum from subject non-plasma blood elements;
b. performing spectral unmixing to determine the weightings of individual basis spectra that contribute to the fluorescence emission spectrum or absorption spectrum; and
c. using the weightings to determine a probability that the subject has a disease associated with abnormal protein aggregation.

As used herein, the term "spectral unmixing" refers to any method by which individual basis spectra are separated from the composite fluorescence emission spectrum or absorption spectrum. In one embodiment, spectral unmixing is performed using the Levenberg-Marquardt algorithm. In another embodiment, spectral unmixing is performed using a linear decomposition algorithm.

In one embodiment, the individual basis spectra are determined from samples of (a) subjects known to have a disease associated with abnormal protein aggregation, (b) healthy control subjects and (c) samples that have not been contacted with the probe. Optionally, the individual basis spectra are determined from samples of non-plasma blood elements such as leukocytes.

In another embodiment, the methods described herein include comparing fluorescence emission spectrum or absorption spectrum to one or more reference fluorescence emission spectra or reference absorption spectra using machine learning, genetic algorithms or principle components analysis. For example, in one embodiment a data set of spectra representative of non-plasma blood elements from subjects with a particular disease state are used to train a genetic algorithm in order to predict the disease state of a subject based on spectral data of non-plasma blood elements, optionally contacted with a probe that binds pathogenic protein aggregates.

A person of skill in the art would readily be able to use the method described above to assess the probability that the subject has a disease associated with abnormal protein aggregation.

In another embodiment, spectral analysis of samples of non-plasma blood elements such as leukocytes may be performed at different time points to detect differences in the maturity of the deposits thereby predicting the stage, severity or rate of progression of the underlying disease.

The presence of pathogenic protein aggregates in non-plasma blood elements can also be detected using an antibody to the pathogenic protein aggregates. In one embodiment, the antibody is an anti-β-amyloid antibody such as 6E10, 4G8, AB5078P, 12F4, AB9234 or OMAB. AB9234 and OMAB are antibodies that bind to β-amyloid oligomers.

While the above methods contemplate detecting aggregates in intact, optionally permeabilized, cells, in another embodiment, aggregates are detected in lysed and/or frozen cells.

In some embodiments, the methods further comprise detecting additional proteins known to be associated with diseases associated with abnormal protein aggregation. For example hyperphosphorylated Tau, prion protein, and/or alphaB-crystallin are optionally detected. Optionally, anti-phospho-Tau is used to detect hyperphosphorylated Tau and 6H4 antibody is used to detect prion protein. Antibodies to pathogenic forms of SOD associated with ALS may also be used.

The probe is optionally a fluorescent probe that binds to the pathogenic protein aggregates. In another embodiment, the probe is a conformationally-sensitive fluorescent probe. Specific examples of conformationally-sensitive fluorescent probes useful for detecting amyloid peptides include, but are not limited to, Congo Red, Congo Red derivatives and bis-styrylbenzenes (e.g. K114, X34, BSB, FSB, IMSB, Chrysamine-G, methoxy-X34, methoxy-X04), thioflavin analogs (thioflavin-T, thioflavin-S, Pittsburgh compound B), thiazine red R, auramine-O and pentameric formyl thiophene acetic acid (p-FTAA) and related luminescent conjugated polythiophenes (LCPs) or luminescent conjugated oligothiophenes (LCOs).

In other embodiments, the probe is an antibody to pathogenic protein aggregates such as an anti β-amyloid antibody, for example, 6E10.

The following non-limiting examples are illustrative of the present disclosure:

### EXAMPLES

### Example 1. Detection of Alzheimer's disease from blood using fluorescence spectroscopy

Many neurological diseases such as AD, Parkinson's, Huntington's and prionopathies are associated with the misfolding and aggregation of proteins from their soluble functional state into highly ordered fibrillar assemblies with high β-sheet content. These pathologically unrelated disorders have in common specific binding to a classic amyloid probe, Congo Red, and are thus called "congophilic" diseases. Conformationally-sensitive fluorescent probes that specifically bind amyloid fibrils have been widely used for the investigation of protein aggregation (Styren et al 2000; Klunk et al 2002; Mathis et al 2002; Aslund et al 2009; Nilsson et al 2005; Hammarstrom et al 2010). Some newer probes have the additional property of detecting soluble oligomeric species, and altering their emission spectra depending on the protein aggregate they bind to, allowing them to identify minute deposits or subtle changes in conformation.

Advanced spectral imaging allows highly quantitative separation of spectra, including subtle variations from amyloid probes binding to different types of aggregates. Using these techniques, an assay was devised for the detection of misfolded proteins (Aβ or others) from brain and blood of an AD mouse model (Oakley et al 2006) and from human AD patients. Spectral signatures of protein aggregates labeled by probe X-34 (a Congo Red derivative) were studied from the CNS and blood.

Strong and specific signals were obtained from AD transgenic mouse brain plaques labeled with X-34, and it was possible to distinguish various states of "maturity" and aggregation within single senile plaques (Fig. 1). This strategy was also applied to human AD patient brains. Here, amyloid plaques could be distinguished from neurofibrillary tangles and autofluorescent lipofuscin in paraffin-embedded sections. β-amyloid signatures were very similar to those from AD mouse brain. Together, these results indicate that the method is capable of sensitive and potentially AD-specific detection of protein misfolding/aggregation.

Immunostaining AD transgenic mouse brain with an anti-Aβ antibody 6E10 showed that senile plaques with typical morphology of 6E10 immuno-positive (Figure 2a). Further, 6E10-positive blood cells were also observed around a blood vessel (Figure 2 a and b), showing that the cells ingested material that is very similar to that found in plaques. Without being bound by theory, it is believed that circulating leukocytes act as "sentinels" with the ability to carry misfolded proteins as a "memory" of their experience after trafficking through the diseased brain.

Whereas analyzing post-mortem brain is the de facto standard for AD diagnosis, it is highly impractical. A non-invasive technique was therefore sought. In support of the hypothesis that leukocytes act as "circulating sentinels", many such cells from AD mouse blood were strongly positive for X-34, with spectral signatures very similar to those from brain plaques, suggesting that it is possible to detect similar "AD-like pathology" in blood (Fig. 3d). Many of these positive leukocytes were also immunopositive for Aβ (Fig. 3a-c), supporting the notion that they had trafficked into the AD brain and/or transited through the brain vasculature, ingested then exported misfolded forms of Aβ within them into the systemic circulation. Probe signal from wild type (littermate control) leukocytes was negligible.

This method was then tested in human AD blood samples. Freshly isolated buffy coat from clinically diagnosed AD and control (healthy, stroke, Creutzfeldt-Jakob disease (CJD)) patients were subjected to a similar analysis. All AD samples showed strongly positive cells with X-34, exhibiting distinctive emission spectra, which were virtually absent from all controls (Fig. 4a-b). Strong AD-specific fluorescence signal was seen only in AD leukocytes (Fig 4c). These results also show that CJD protein aggregates are not detectable using the probe X-34 with the basis spectra used for this analysis (specifically selected to detect AD), and show that different fluorescent probes and/or detection of different specific emission spectra, unique for CJD and not for AD, would be necessary to detect the unique putative misfolded proteins associated with CJD. Lastly, all X-34-positive leukocytes co-labeled with anti-Aβ (Fig. 5), indicating that as in AD mouse, it is possible to detect "AD-like pathology" in circulating human blood leukocytes.

Taken together, these observations show that it is possible to detect AD pathology in circulating leukocytes. Such peripheral abnormalities reflect relevant pathology in AD brain, and thus constitute a reliable test for AD.

### Example 2: Detecting protein aggregates in leukocytes using probe K114

Fluorescent probe K114, an amyloid-specific dye and an analogue of Congo Red, was used to detect protein aggregates in human leukocytes. In addition, fluorescence signals from the labeled leukocytes were processed using algorithms (for example, algorithms based on the Levenberg-Marquardt damped least-squares non-linear curve fitting algorithm) to separate disease-specific signals from background and auto fluorescence emission. A numerical score based on fluorescence emissions was calculated reflecting the probability that a test cell sample originated from a patient with a disease associated with abnormal protein aggregation.

### Protocol

Blood was drawn from patients in an EDTA tube and placed on ice. The buffy coat (leukocytes) was isolated using standard techniques. Briefly, blood samples were centrifuged at 1,700 RCF at room temperature and the concentrated leukocyte band (middle white layer) was removed. Contaminating red blood cells were lysed by ACK lysing buffer for 3-5 min at room temperature and leukocyte samples were washed with PBS.

Leukocytes were fixed in a 10% neutralized buffered formalin. A small aliquot of fixed leukocytes were placed on a slide glass and dried. The dried sample was stained with fluorescent probe K114 or X-34.

The probe was washed with buffer, coverslipped with water-based mounting media and imaged on a laser scanning microscope.

Imaging was performed either with continuous-wave laser illumination (1-photon excitation with confocal detection) or with 2-photon excitation, both at various and multiple wavelengths. Optionally, multiphoton excitation may also be used.

Several images of several hundred cells each were acquired using a spectral imaging system that acquired 32 channels of spectrally-resolved data spanning 400-750 nm (typical).

Images of cells were analyzed for fluorescent inclusions (which are present in both control and AD/MCI (Mild Cognitive Impairment) samples) and for increased fluorescence in the leukocyte cytoplasm. These inclusions were classified by size, and distinct fluorescent spectra extracted manually as an initial step. These "basis spectra" represent the various emitters in the sample: autofluorescence, laser backscatter artifact, several distinct emissions from each fluorescent probe. These spectra differ according to excitation wavelength, and are re-determined for each wavelength (typically 2 different excitation wavelengths were used).

Using these basis spectra, the combined fluorescence in the combined images was processed to determine the relative weighting/contribution of each basis spectrum for each sample, yielding a numerical coefficient for each basis spectrum. The software was based in part on the Levenberg-Marquardt damped least-squares non-linear curve fitting algorithm.

A ratio of coefficients was used to compute a "disease index" for each category: control, MCI (Mild Cognitive Impairment), AD, other neurological disorders. Different disorders e.g. CJD, BSE require different fluorescent probes and/or different basis spectra to detect unique putative misfolded proteins.

### Results

AD-specific signals were observed in leukocytes from AD patients labeled with K114 (Fig. 6a). The emission spectra of healthy cells labeled with K114 were distinguishable with AD cells labeled with K114 (Fig. 6b).

Figure 7 is a simplified composite index that reflects the probability that a subject has either AD or Mild Cognitive Impairment (MCI) ("ADR index" ie "AD related index"). Leukocytes were sampled from healthy controls (Healthy cont), stroke patients without AD/MCI (Stroke), patients with "other neurological disorders" (OND) and patients with Alzheimer's disease (AD), Mild Cognitive Impairment (MCI) and cerebral amyloid angiopathy (CAA). The OND patient had CJD.

While all the non-ADR controls (Healthy Control, Stroke and CJD) were negative, all of the ADR patients (AD + MCI) were positive. In addition, three out of four of the cerebral amyloid angiopathy (CAA) patients were positive. This data shows that the method clearly identifies patients with Alzheimer's disease and related disorders.

### Example 3. Presence of amyloids in the brain of an MS patient

It is generally believed that AD is a neurodegenerative disorder with secondary innate inflammation, but the thinking for multiple sclerosis (MS) is unclear. Initially, evidence was strong in favor of a primary autoimmune disorder with the resulting inflammatory assault driving demyelination and cortical atrophy. However, recent data from detailed pathological examination, and experience with potent anti-inflammatory therapies, cast doubt on this conclusion (Stys et al 2012).

Therefore, the possibility exists that MS may also be a primary degenerative disorder, with the unusually prominent inflammation a secondary response. What then might be at the root of MS? Pathology of later chronic MS (where the inflammatory reaction is less prominent) indicates that demyelinating lesions expand with time (possibly propagating from discrete foci within plaques?), and the prominent periventricular and cortical pathology suggests that some soluble factor (possibly circulating in the CSF?) may be responsible. Amyloids have this property in that they may propagate and misfold additional proteins, circulate as toxic soluble species (e.g. Aβ oligomers in AD), and later, condense into insoluble aggregates. Fig. 8 shows human MS brain stained with Congo Red, an amyloid probe. These data suggest that chronic MS plaques have deposits of amyloid. These deposits may be a primary feature of MS causing myelin and neuronal damage, as Aβ is thought to do in AD, or they may be reactive to chronic bouts of inflammation. Regardless, the presence of amyloid plaques in MS brain indicates that similar to AD, circulating leukocytes of MS patients are also likely to carry detectable amyloid aggregates.

### Example 4. Larger scale study: Blood test for AD using fluorescence spectroscopy

Blood samples from 30 AD and 30 age-matched healthy controls, as well as other non-AD neurodegenerative disorders, are obtained. Buffy coat is isolated and leukocytes labeled with conformationally-sensitive probes. Spectral 2-photon and 1-photon images are acquired and fluorescence signals from leukocytes processed using algorithms to separate AD-specific from background and autofluorescence emissions. The spectral signatures of the leukocyte amyloid probe fluorescence from AD subjects are virtually identical to that from human and mouse senile plaque, strongly suggesting that certain leukocyte subsets (≈1-2%) contain material that is similar to that found in cerebral plaques. Further, these probe-positive leukocytes co-label strongly for anti-Aβ antibody, consistent with the understanding certain leukocytes trafficked through the brain (microvasculature and/or parenchyma), were exposed to an Aβ-rich milieu, and brought with them a "memory" of this exposure that is detectable by the present methods. A numerical score based on the different fluorescence emissions is then calculated reflecting the probability that the sample originated from an AD patient. In select subjects, post-mortem neuropathological examination is performed to unequivocally confirm the diagnosis.

There is evidence that "activation" of leukocytes e.g. by infection, also increases probe fluorescence, though the spectral signatures differ. Therefore human subjects with various infections, auto-immune diseases, and lymphoproliferative disorders (leukemia, lymphoma) are also included to ensure the specificity of the method.

There is also evidence that 1-photon fluorescence excitation (confocal) can be used, greatly simplifying the technique and opening the way for future development of a relatively inexpensive high-throughput instrument.

### Example 5. Blood test for bovine spongiform encephalopathy (BSE) using fluorescence spectroscopy

Using blood from scrapie-infected and healthy mice, red blood cells (RBCs) were removed by osmotic shock, and non-RBC blood elements separated. Cells were stained with conformationally-sensitive amyloid probes. Strongly positive signal was only seen in scrapie blood elements, and no signal was found in uninfected mice. Moreover the spectral signature from the scrapie-infected blood samples was identical to scrapie spleen in proteinase K-resistant, anti-PrP-positive regions.

The method was then tested in experimental BSE samples. Frozen isolated buffy coat from neuropathologically proven BSE and non-BSE controls, together with healthy cows and those with other diseases (e.g. polio, brain abscess) were subjected to similar analysis using 2 different conformationally-sensitive amyloid probes. Both BSE samples were strongly positive, and all other samples (both healthy control and infected non-BSE cattle) were unequivocally negative. This data shows that the spectral fluorescence methods described herein are capable of reliably detecting markers in the blood present only in BSE-afflicted animals.

### Example 6. Spectral fluorescence analysis of blood and brain sections from transgenic mice with Alzheimer's disease

Studies were performed on an AD transgenic mouse strain (5XFAD, Oakley et al, 2006) comparing blood signatures of AD obtained using the spectral fluorescence methods described herein with brain AD pathology. The advantage of this approach, unlike with human subjects, is that it is possible to know precisely the extent of amyloid brain pathology at any given age.

AD index was calculated from mouse blood by selecting fluorescence spectra from leukocytes stained with K-114 and imaged on a spectral confocal microscope. Excitation wavelengths used include 375, 405, 445 and 457 nm, however other wavelengths including but not limited to wavelengths intermediate to these values can also be used. AD and healthy age-matched wild type mice were used. These spectra were then used to calculate coefficients to indicate how close aggregate spectra from many cells/image approximate diseased vs. normal spectra. The coefficient representing the AD component was then multiplied by the mean pixel intensity to obtain the AD index for blood.

Mouse brain sections were formalin-fixed and stained with K-114. Representative spectral images of these sections were acquired, and brain regions were selected based on fluorescence intensity thresholds. Selected pixels were then aggregated (pixels in the 2D image summed and collapsed into a single spectral vector consisting of intensity vs. wavelength) and processed using AD and control spectra as for blood above. The resulting coefficients were multiplied by the mean pixel intensity to obtain the AD index for brain.

Results are shown in Figure 9. As previously published (Oakley et al, 2006), AD plaque pathology begins at about 2 months, increasing rapidly with age. Using the spectral fluorescence assay described herein, blood from the same subjects becomes positive at 2 months, at the same age as the earliest start of brain pathology. Wild type (WT) brain and blood remain negative. This provides further support to the sensitivity of the methods described herein as being able to detect very early disease in the blood.

### Example 7. Spectral fluorescence analysis of blood and cerebrospinal fluid from a human subject using K-114

The use of human subjects in Alzheimer's disease (AD) research is complicated by the fact that it is difficult to be certain of the diagnosis or stage of the disease from ante-mortem human samples. In order to investigate whether the spectral fluoresce methods described herein correspond with other detection methods in human subjects, investigations were done by testing blood samples in parallel with cerebrospinal fluid (CSF) samples and analyzing the CSF using the currently accepted clinical standard (CSF levels of Aβ(1-42) and tau/phospho-tau tested using Athena Diagnostics ADmark® Phospho-Tau/Totat-Tau/Aβ42 CSF assay) in order to determine whether a prospective subject has AD.

2D spectral confocal images were obtained from smears of leukocytes stained with K-114 from a sample from a subject that was positively identified as having AD by detecting CSF levels of Aβ(1-42) and tau/phospho-tau as well as from a sample from a healthy control. K-114 emission was isolated based on signal intensity, integrated, and spectra extracted from AD and HC samples. Cellular regions that did not exhibit any K-114 emission were masked to eliminate background noise. Normalized spectra for each sample are shown in Figure 10. The fluorescent spectrum obtained from the subject with AD is easily distinguished from the healthy control and is significantly blue-shifted towards shorter wavelengths.

### Example 8. Detection of Aβ(1-42) and pathogenic protein aggregates in sample of peripheral leukocytes from subjects with Alzheimer's disease and healthy controls

Samples of peripheral leukocytes from subjects with Alzheimer's disease and healthy controls were stained using a detectable antibody for Aβ(1-42) (Millipore AB5078P) resulting in a green fluorescent signal and were also stained with a probe that binds to pathogenic protein aggregates (K114) resulting in a red fluorescent signal.

As shown in Figure 11, both healthy control (Figure 11A) and AD samples (Figure 11B) exhibit Aβ1-42 immunostaining in leukocytes. The mean fluorescent staining intensity in both samples was roughly equal (Figure 11C). However, Figure 11B shows a much higher red intensity, which represents AD-specific spectral emission from the conformationally selective amyloid probe co-stained together with the antibody, indicating the presence of pathogenic protein aggregates. There is very little AD-specific red signal in the control sample. This underscores the observation that simple immunodetection of Aβ1-42 in peripheral leukocytes is unable by itself to reliably distinguish between healthy controls and AD. Analysis of the shape of the spectra from leukocytes stained with fluorescent probes that selectively bind pathogenic protein aggregates provides significantly more information and facilitates distinguishing normal subjects from subjects with AD compared to simply analyzing fluorescent intensity at a particular wavelength. Likewise, analysis and comparison of spectra at multiple wavelengths is expected to facilitate detecting other diseases associated with abnormal protein aggregation.

### Example 9. Spectral fluorescence analysis of blood from a subject with traumatic brain injury

Traumatic brain injury (TBI) is a major risk factor for AD, and studies have shown that amyloid plaque deposition may be significant soon after injury. Here, the inventors sought to apply a similar approach used for assaying blood from subjects suspected of having AD to assaying blood from subjects with TBI in order to detect pathogenic protein aggregates soon after brain injury as a means to estimate the biochemical amyloid burden in the brain.

Blood samples were obtained from a male subject who suffered a severe closed head injury and TBI 5 days after the injury while the subject was still in a coma.

Figure 12 shows the results of spectral analysis of a sample of leukocytes from the subject with TBI 5 days after the injury along with spectra obtained from a healthy control and from a subject with Alzheimer's disease. Spectra were obtained from blood leukocytes processed and stained using K-114 as the probe. 2-D imaging was performed as set out in Example 7. Spectra were all obtained using a 375 nm excitation wavelength. As shown in Figure 12, the spectrum from the subject with TBI appears red-shifted relative to the others, and clearly falls outside the normal range.

While the present disclosure has been described with reference to what are presently considered to be the preferred examples, it is to be understood that the disclosure is not limited to the disclosed examples. To the contrary, the disclosure is intended to cover various modifications and equivalent arrangements included within the scope of the appended claims.

### References

1. Barnes, D. E. & Yaffe, K. The projected effect of risk factor reduction on Alzheimer's disease prevalence. Lancet Neurol 10, 819-828 (2011).
2. Selkoe, D. J. Alzheimer's disease: genes, proteins, and therapy. Physiol Rev 81, 741-766 (2001).
3. Britschgi, M. & Wyss-Coray, T. Systemic and acquired immune responses in Alzheimer's disease. Int Rev Neurobiol 82, 205-233 (2007).
4. de la Torre, J. C. Alzheimer disease as a vascular disorder: nosological evidence. Stroke 33, 1152-1162 (2002).
5. Deane, R. & Zlokovic, B. V. Role of the blood-brain barrier in the pathogenesis of Alzheimer's disease. Curr Alzheimer Res 4, 191-197 (2007).
6. Nagababu, E., Usatyuk, P. V., Enika, D., Natarajan, V. & Rifkind, J. M. Vascular endothelial barrier dysfunction mediated by amyloid-beta proteins. J Alzheimers Dis 17, 845-854 (2009).
7. Farkas, I. G. et al. Beta-amyloid peptide-induced blood-brain barrier disruption facilitates T-cell entry into the rat brain. Acta Histochem 105, 115 125 (2003).
8. Majumdar, A. et al. Degradation of fibrillar forms of Alzheimer's amyloid betapeptide by macrophages. Neurobiol Aging 29, 707-715 (2008).
9. Mildner, A. et al. Microglia in the adult brain arise from Ly-6ChiCCR2+ monocytes only under defined host conditions. Nat Neurosci 10, 1544-1553 (2007).
10. Simard, A. R., Soulet, D., Gowing, G., Julien, J. P. & Rivest, S. Bone marrowderived microglia play a critical role in restricting senile plaque formation in Alzheimer's disease. Neuron 49, 489-502 (2006).
11. Stamatovic, S. M. et al. Monocyte chemoattractant protein-1 regulation of bloodbrain barrier permeability. J Cereb Blood Flow Metab 25, 593-606 (2005).
12. McKhann, G. M. et al. The diagnosis of dementia due to Alzheimer's disease: recommendations from the National Institute on Aging-Alzheimer's Association workgroups on diagnostic guidelines for Alzheimer's disease. Alzheimers Dement 7, 263-269 (2011).
13. Styren, S. D., Hamilton, R. L., Styren, G. C. & Klunk, W. E. X-34, a fluorescent derivative of Congo red: a novel histochemical stain for Alzheimer's disease pathology. J Histochem Cytochem 48, 1223-1232 (2000).
14. Klunk, W. E. et al. Imaging Abeta plaques in living transgenic mice with multiphoton microscopy and methoxy-X04, a systemically administered Congo red derivative. J Neuropathol Exp Neurol 61, 797-805 (2002).
15. Mathis, C. A. et al. A lipophilic thioflavin-T derivative for positron emission tomography (PET) imaging of amyloid in brain. Bioorg Med Chem Lett 12, 295-298 (2002).
16. Aslund, A. et al. Novel pentameric thiophene derivatives for in vitro and in vivo optical imaging of a plethora of protein aggregates in cerebral amyloidoses. ACS Chem Biol 4, 673-684 (2009).
17. Nilsson, K. P., Herland, A., Hammarstrom, P. & Inganas, O. Conjugated polyelectrolytes: conformation-sensitive optical probes for detection of amyloid fibril formation. Biochemistry 44, 3718-3724 (2005).
18. Hammarstrom, P. et al. A fluorescent pentameric thiophene derivative detects in vitro-formed prefibrillar protein aggregates. Biochemistry 49, 6838-6845 (2010).
19. Oakley, H. et al. Intraneuronal beta-amyloid aggregates, neurodegeneration, and neuron loss in transgenic mice with five familial Alzheimer's disease mutations: potential factors in amyloid plaque formation. J Neurosci 26, 10129-10140 (2006).
20. Caumartin, J., Lemaoult, J. & Carosella, E. D. Intercellular exchanges of membrane patches (trogocytosis) highlight the next level of immune plasticity. Transpl Immunol 17, 20-22 (2006).
21. Thambisetty, M. & Lovestone, S. Blood-based biomarkers of Alzheimer's disease: challenging but feasible. Biomark Med 4, 65-79 (2010).
22. van Rossum, I. A. et al. Injury markers predict time to dementia in subjects with MCI and amyloid pathology. Neurology (2012).
23. Senanarong, V. et al. Alzheimer's disease dementia as the diagnosis best supported by the cerebrospinal fluid biomarkers: difference in cut-off levels from thai experience. Int J Alzheimers Dis 2012, 212063 (2012).
24. Jack, C. R. J. Alzheimer disease: new concepts on its neurobiology and the clinical role imaging will play. Radiology 263, 344-361 (2012).
25. Matsuda, H. & Imabayashi, E. Molecular neuroimaging in Alzheimer's disease. Neuroimaging Clin N Am 22, 57-65, viii (2012).
26. Nordstedt, C. et al. Human neutrophil phagocytic granules contain a truncated soluble form of the Alzheimer beta/A4 amyloid precursor protein (APP). J Biol Chem 269, 9805-9810 (1994).
27. Styren, S. D. et al. X-34, A Fluorescent Derivative of Congo Red: A Novel Histochemical Stain for Alzheimer's Disease Pathology. J Histochem Cytochem September 2000 vol. 48 no. 9 1223-1232
28. Crystal, A.S. et al. A comparison of amyloid fibrillogenesis using the novel fluorescent compound K114. J Neurochem. 2003 Sep;86(6): 1359-68.
29. Vicart, P. et al. A missense mutation in the alphaB-crystallin chaperone gene causes a desmin-related myopathy. Nature Genetics 20, 92 - 95 (1998)
30. Sharma MC, Goebel HH. Protein aggregate myopathies. Neurol India. 2005 Sep;53(3):273-9.
31. Stys PK, et al. Will the real multiple sclerosis please stand up? Nat Rev Neurosci. 2012 Jul; 13(7):507-14.

## Claims

1. A method of detecting a disease associated with abnormal protein aggregation in a subject, comprising:
contacting one or more non-plasma blood elements obtained from the subject with a conformationally-sensitive probe that binds to pathogenic protein aggregates, wherein the non-plasma blood elements are selected from red blood cells, microvesicles, leukocytes and platelets,
generating a fluorescence emission spectra or absorption spectra of the probe bound to the pathogenic protein aggregates, wherein the emission spectra or the absorption spectra of the conformationally-sensitive probe changes when the probe is bound to the pathogenic protein aggregate, and
comparing the fluorescence emission spectra or absorption spectra of the probe contacted with the non-plasma blood elements to one or more reference fluorescence emission spectra or absorption spectra, wherein the reference non-plasma blood elements are from a reference subject who has a disease associated with abnormal protein aggregation and correspondence between the fluorescence emission spectrum or absorption spectra and the one or more reference fluorescence emission spectra or absorption spectra is indicative that the subject has a disease associated with abnormal protein aggregation, wherein the disease associated with abnormal protein aggregation is Alzheimer's disease, mild cognitive impairment, cerebral amyloid angiopathy, brain trauma, traumatic brain injury, bovine spongiform encephalopathy or multiple sclerosis.

2. The method of claim 1, wherein the one or more non-plasma blood elements are selected from red blood cells, leukocytes and platelets.

3. The method of claim 1 or 2, wherein the probe is K114, Congo Red, X34, BSB, FSB, IMSB, Chrysamine-G, methoxy-X34, methoxy-X04, thioflavin-T, thioflavin-S, Pittsburgh compound B, thiazine red R, auramine-O, p-FTAA or a luminescent conjugated polythiophene (LCP) or luminescent conjugated oligothiophene (LCO) related to p-FTAA.

4. The method of any one of claims 1 to 3, wherein generating the fluorescence emission spectra of the probe comprises detecting the intensity of the fluorescence emission signal at two or more wavelengths, optionally elicited using 1- or multi-photon excitation at one or more excitation wavelengths.

5. The method of any one of claims 1 to 4, wherein the method comprises:
a. performing spectral unmixing to determine the weightings of individual basis spectra that contribute to the fluorescence emission spectrum or absorption spectrum; and
b. using the weightings to determine a probability that the subject has a disease associated with abnormal protein aggregation.

6. The method of claim 5, wherein the individual basis spectra are determined from (a) samples of subjects known to have a disease associated with abnormal protein aggregation, (b) samples of healthy control subjects and/or (c) samples that have not been contacted with the probe.

7. The method of claim 5 or 6, wherein the spectral unmixing is performed using linear algebraic methods, optionally using non-linear algebraic methods such as using the Levenberg-Marquardt algorithm.

8. The method of any one of claims 4 to 7, wherein comparing the fluorescence emission spectrum or absorption spectrum to one or more reference fluorescence emission spectra or reference absorption spectra comprises machine learning, genetic algorithms or principle components analysis.

9. The method of any one of claims 1 to 8, wherein the disease associated with abnormal protein aggregation is Alzheimer's disease, brain trauma, traumatic brain injury or multiple sclerosis.

10. The method of any one of claims 1 to 9, wherein the pathogenic protein aggregates comprise amyloid proteins, optionally β-amyloid, tau protein, hyperphosphorylated tau protein (pTau), prion protein, and/or αB-crystallin (CRYAB).

11. The method of any one of claims 1 to 8, wherein the pathogenic protein aggregates comprise β-amyloids and the disease is Alzheimer's disease.

12. The method of any one of claims 1 to 11, wherein the method comprises generating two or more fluorescence emission spectra at two or more excitation wavelengths, optionally wherein at least one of the excitation wavelengths is in the near-ultraviolet, optionally 200nm to 400 nm, or wherein at least one of the excitation wavelengths is from 300nm to 500 nm, optionally 375 nm or 445 nm.

13. The method of any one of claims 1 to 12, wherein detecting the fluorescence of the probe bound to the pathogenic protein aggregates comprises measuring the intensity of the fluorescence emission at one or more wavelengths between 200 nm and 2000nm or detecting the absorbance of the probe bound to the pathogenic protein aggregates comprises measuring the absorbance of light at one or more wavelengths between 200nm and 2000nm.

14. The method of any one of claims 1 to 13, wherein generating the fluorescence emission spectra or absorption spectra comprises measuring the fluorescence or absorbance of the probe bound to the pathogenic protein aggregates in all or part of a 2-dimensional field comprising a sample of leukocytes.

15. The method of any one of claims 1 to 13, wherein generating the fluorescence emission spectra or absorption spectra comprises using a non-imaging spectrometer.

## Patentansprüche

1. Verfahren zum Feststellen einer Krankheit, die mit anomaler Proteinaggregation in einer Person verbunden ist, umfassend:
Kontaktieren von einem oder mehreren Nicht-Plasma-Blutelementen, die von der Person mit einer konformationsempfindlichen Sonde gewonnen wurde, die sich an die pathogenen Proteinaggregate bindet, wobei die Nicht-Plasma-Blutelemente aus roten Blutzellen, Mikrovesikeln, Leukozyten und Plättchen ausgewählt werden,
Erzeugen eines Fluoreszenz-Emissionsspektrums oder Absorptionsspektrums der Sonde, die an die pathogenen Proteinaggregate gebunden ist, wobei die Emissionsspektren oder die Absorptionsspektren der konfirmationsempfindlichen Sonde sich ändern, wenn die Sonde an das pathogene Proteinaggregat gebunden ist, und
Vergleichen der Fluoreszenzemissionsspektren oder Absorptionsspektren der Sonde, die mit den Nicht-Plasma-Blutelementen in Kontakt gebracht wurde, mit einem oder mehreren Referenz-Fluoreszenz-Emissionsspektren oder Absorptionsspektren, wobei die Referenz-Nicht-Plasma-Blutelemente von einer Referenzperson sind, die eine Krankheit hat, die mit anomaler Proteinaggregation verbunden ist, und eine Übereinstimmung zwischen dem Fluoreszenz-Emissionsspektrum oder Absorptionsspektren und dem einen oder den mehreren Referenz-Fluoreszenz-Emissionsspektren oder Absorptionsspektren ist kennzeichnend dafür, dass die Person eine Krankheit hat, die mit anomaler Proteinaggregation verbunden ist, wobei die Krankheit, die mit anomaler Proteinaggregation verbunden ist, die Alzheimer-Krankheit, leichte kognitive Beeinträchtigung, zerebrale Amyloid-Angiopathie, Hirntrauma, traumatische Hirnverletzung, Bovine Spongiforme Enzephalopathie oder Multiple Sklerose ist.

2. Verfahren nach Anspruch 1, wobei das eine oder die mehreren Nicht-Plasma-Blutelemente aus roten Blutzellen, Leukozyten und Plättchen ausgewählt werden.

3. Verfahren nach Anspruch 1 oder 2, wobei die Sonde K114, Kongorot, X34, BSB, FSB, IMSB, Chrysamine-G, Methoxy-X34, Methoxy-X04, Thioflavin-T, Thioflavin-S, Pittsburgh Compound B, Thiazin-Rot R, Auramin-O, p-FTAA oder ein lumineszentes konjugiertes Polythiophen (LCP) oder lumineszentes konjugiertes Oligothiophen (LCO) ist, das mit p-FTAA verwandt ist.

4. Verfahren nach einem der Ansprüche 1 bis 3, wobei das Erzeugen der Fluoreszenz-Emissionsspektren der Sonde das Feststellen der Intensität des Fluoreszenz-Emissionssignals bei zwei oder mehr Wellenlängen umfasst, die optional unter Verwendung von 1- oder Multi-Photonen-Anregung bei einer oder mehreren Anregungswellenlängen angeregt wurden.

5. Verfahren nach einem der Ansprüche 1 bis 4, wobei das Verfahren umfasst:
a. Ausführen der spektralen Entmischung, um die Gewichtungen der einzelnen Basisspektren zu bestimmen, die zum Fluoreszenz-Emissionsspektrum oder Absorptionsspektrum beitragen; und
b. unter Verwendung der Gewichtungen, Bestimmen einer Wahrscheinlichkeit, dass die Person eine Krankheit hat, die mit anomaler Proteinaggregation verbunden ist.

6. Verfahren nach Anspruch 5, wobei die individuellen Grundspektren bestimmt werden aus (a) Proben von Personen, von denen bekannt ist, dass sie eine Krankheit haben, die mit anomaler Proteinaggregation verbunden ist, (b) Proben von gesunden Kontrollpersonen und/oder (c) Proben, die nicht mit der Sonde in Kontakt gebracht worden sind.

7. Verfahren nach Anspruch 5 oder 6, wobei die spektrale Entmischung unter Verwendung von linearen algebraischen Verfahren, optional unter Verwendung von nichtlineare algebraische Verfahren wie zum Beispiel die Verwendung des Levenberg-Marquardt-Algorithmus ausgeführt wird.

8. Verfahren nach einem der Ansprüche 4 bis 7, wobei das Vergleichen des Fluoreszenz-Emissionsspektrums oder Absorptionsspektrums mit einem oder mehreren Referenz-Fluoreszenz-Emissionsspektren oder Referenz-Absorptionsspektren das Maschinenlernen, genetische Algorithmen oder Hauptkomponentenanalyse umfasst.

9. Verfahren nach einem der Ansprüche 1 bis 8, wobei die Krankheit, die mit anomaler Proteinaggregation verbunden ist, die Alzheimer-Krankheit, Hirntrauma, traumatische Hirnverletzung oder Multiple Sklerose ist.

10. Verfahren nach einem der Ansprüche 1 bis 9, wobei die pathogenen Proteinaggregate Amyloidproteine, optional β-Amyloid, Tau-Protein, hyperphosphoryliertes Tau-Protein (pTau), Prionprotein und/oder αB-Crystallin (CRYAB) umfassen.

11. Verfahren nach einem der Ansprüche 1 bis 8, wobei die pathogenen Proteinaggregate β-Amyloide umfassen, und die Krankheit ist die Alzheimer-Krankheit.

12. Verfahren nach einem der Ansprüche 1 bis 11, wobei das Verfahren das Erzeugen von zwei oder mehr Fluoreszenz-Emissionsspektren bei zwei oder mehr Anregungswellenlängen umfasst, wobei optional mindestens eine der Anregungswellenlängen im Nahultraviolett liegt, optional 200 nm bis 400 nm, oder wobei mindestens eine der Anregungswellenlängen von 300 nm bis 500 nm reicht, optional 375 nm oder 445 nm.

13. Verfahren nach einem der Ansprüche 1 bis 12, wobei das Feststellen der Fluoreszenz der Sonde, die an die pathogenen Proteinaggregate gebunden ist, das Messen der Intensität der Fluoreszenzemission bei einer oder mehreren Wellenlängen zwischen 200 nm und 2000 nm umfasst, oder das Feststellen der Absorption der Sonde, die an die pathogenen Proteinaggregate gebunden ist, das Messen der Absorption von Licht bei einer oder mehreren Wellenlängen zwischen 200 nm und 2000 nm umfasst.

14. Verfahren nach einem der Ansprüche 1 bis 13, wobei das Erzeugen der Fluoreszenz-Emissionsspektren oder Absorptionsspektren das Messen der Fluoreszenz oder Absorption der Sonde, die an die pathogenen Proteinaggregate gebunden ist, im ganzen oder in einem Teil eines zweidimensionalen Feldes umfasst, das eine Probe von Leukozyten enthält.

15. Verfahren nach einem der Ansprüche 1 bis 13, wobei das Erzeugen der Fluoreszenz-Emissionsspektren oder Absorptionsspektren die Verwendung eines nicht-bilderzeugenden Spektrometers umfasst.

## Revendications

1. Procédé pour détecter une maladie associée à une agrégation protéique anormale chez un sujet, comprenant :
la mise en contact d'un ou plusieurs éléments sanguins non plasmatiques obtenus auprès du sujet avec une sonde sensible à la conformation qui se lie à des agrégats protéiques pathogènes, dans lequel les éléments sanguins non plasmatiques sont choisis parmi les globules rouges, les microvésicules, les leucocytes et les plaquettes,
la génération de spectres d'absorption ou de spectres d'émission de fluorescence de la sonde liée aux agrégats protéiques pathogènes, dans lequel les spectres d'absorption ou les spectres d'émission de la sonde sensible à la conformation changent quand la sonde est liée à l'agrégat protéique pathogène, et
la comparaison des spectres d'absorption ou des spectres d'émission de fluorescence de la sonde venue au contact des éléments sanguins non plasmatiques avec un ou plusieurs spectres d'absorption ou spectres d'émission de fluorescence de référence, dans lequel les éléments sanguins non plasmatiques de référence proviennent d'un sujet de référence qui est atteint d'une maladie associée à une agrégation protéique anormale et une correspondance entre les spectres d'absorption ou spectres d'émission de fluorescence et le ou les spectres d'absorption ou spectres d'émission de fluorescence de référence est indicative du fait que le sujet est atteint d'une maladie associée à une agrégation protéique anormale, dans lequel la maladie associée à une agrégation protéique anormale est la maladie d'Alzheimer, un trouble cognitif léger, une angiopathie amyloïde cérébrale, un traumatisme cérébral, une lésion cérébrale traumatique, l'encéphalopathie spongiforme bovine, ou la sclérose en plaques.

2. Procédé selon la revendication 1, dans lequel le ou les éléments sanguins non plasmatiques sont choisis parmi les globules rouges, les leucocytes et les plaquettes.

3. Procédé selon la revendication 1 ou 2, dans lequel la sonde est K114, le rouge Congo, X34, BSB, FSB, IMSB, la chrysamine G, le méthoxy-X34, le méthoxy-X04, la thioflavine T, la thioflavine S, le composé Pittsburgh B, le rouge de thiazine R, l'auramine-O, p-FTAA ou un polythiophène conjugué luminescent (LCP) ou oligothiophène conjugué luminescent LCO) apparenté à p-FTAA.

4. Procédé selon l'une quelconque des revendications 1 à 3, dans lequel la génération des spectres d'émission de fluorescence de la sonde comprend la détection de l'intensité du signal d'émission de fluorescence à deux ou plus de deux longueurs d'onde, éventuellement déclenchées par utilisation d'une excitation par un seul photon ou par photons multiples à une ou plusieurs longueurs d'onde d'excitation.

5. Procédé selon l'une quelconque des revendications 1 à 4, dans lequel le procédé comprend :
a. la mise en oeuvre d'une ségrégation spectrale pour déterminer les pondérations des spectres de base individuels qui contribuent au spectre d'absorption ou au spectre d'émission de fluorescence ; et
b. l'utilisation des pondérations pour déterminer une probabilité que le sujet soit atteint d'une maladie associée à une agrégation protéique anormale.

6. Procédé selon la revendication 5, dans lequel les spectres de base individuels sont déterminés à partir (a) d'échantillons de sujets connus pour être atteints d'une maladie associée à une agrégation protéique anormale, (b) d'échantillons de sujets témoins en bonne santé et/ou (c) d'échantillons qui n'ont pas été mis en contact avec la sonde.

7. Procédé selon la revendication 5 ou 6, dans lequel la ségrégation spectrale est effectuée par utilisation de procédés algébriques linéaires, éventuellement par utilisation de procédés algébriques non linéaires, comme par utilisation de l'algorithme de Levenberg-Marquardt.

8. Procédé selon l'une quelconque des revendications 4 à 7, dans lequel la comparaison du spectre d'absorption ou du spectre d'émission de fluorescence avec un ou plusieurs spectres d'absorption de référence ou spectres d'émission de fluorescence de référence comprend un apprentissage machine, des algorithmes génétiques ou une analyse de composantes principales.

9. Procédé selon l'une quelconque des revendications 1 à 8, dans lequel la maladie associée à une agrégation protéique anormale est la maladie d'Alzheimer, un traumatisme cérébral, une lésion cérébrale traumatique ou la sclérose en plaques.

10. Procédé selon l'une quelconque des revendications 1 à 9, dans lequel les agrégats protéiques pathogènes comprennent des protéines amyloïdes, éventuellement la protéine β-amyloïde, la protéine tau, la protéine tau hyperphosphorylée (pTau), la protéine de prion, et/ou la protéine αB-cristalline (CRYAB).

11. Procédé selon l'une quelconque des revendications 1 à 8, dans lequel les agrégats protéiques pathogènes comprennent des β-amyloïdes et la maladie est la maladie d'Alzheimer.

12. Procédé selon l'une quelconque des revendications 1 à 11, dans lequel le procédé comprend la génération de deux ou plus de deux spectres d'émission de fluorescence à deux ou plus de deux longueurs d'onde d'excitation, éventuellement dans lequel au moins l'une des longueurs d'onde d'excitation est dans le proche ultraviolet, éventuellement de 200 nm à 400 nm, ou dans lequel au moins l'une des longueurs d'onde d'excitation est de 300 nm à 500 nm, éventuellement de 375 nm ou 445 nm.

13. Procédé selon l'une quelconque des revendications 1 à 12, dans lequel la détection de la fluorescence de la sonde liée aux agrégats protéiques pathogènes comprend la mesure de l'intensité de l'émission de fluorescence à une ou plusieurs longueurs d'onde entre 200 nm et 2000 nm, ou la détection de l'absorbance de la sonde liée aux agrégats protéiques pathogènes comprend la mesure de l'absorbance d'une lumière à une ou plusieurs longueurs d'onde entre 200 nm et 2000 nm.

14. Procédé selon l'une quelconque des revendications 1 à 13, dans lequel la génération des spectres d'absorption ou spectres d'émission de fluorescence comprend la mesure de l'absorbance ou de la fluorescence de la sonde liée aux agrégats protéiques pathogènes dans tout ou partie d'un champ bidimensionnel comprenant un échantillon de leucocytes.

15. Procédé selon l'une quelconque des revendications 1 à 13, dans lequel la génération des spectres d'absorption ou spectres d'émission de fluorescence comprend l'utilisation d'un spectromètre sans imagerie.
